# FASCICULE DE BREVET EUROPEEN

(11) **EP 0 478 446 B1**
(45) Date de publication et mention de la délivrance du brevet: **19.11.1997**
(21) Numéro de dépôt: 91402546.5
(22) Date de dépôt: 25.09.1991
(51) Int. Cl.: C07D 277/68, A61K 31/425, C07D 263/58, C07D 265/36, C07D 279/16, C07D 417/12, C07D 413/12, C07D 491/04, A61K 31/42, A61K 31/535, A61K 31/54

(54) **Nouvelles amines alkyl hétérocycliques, leur procédé de préparation et les compositions pharmaceutiques qui les contiennent**
Heterozyklische Alkylamine, Verfahren zu ihrer Herstellung und pharmazeutische Zusammensetzungen die sie enthalten
Heterocyclic alkylamines, process for their preparation and pharmaceutical compositions containing them

(30) Priorité: 26.09.1990 FR 9011866
(43) Date de publication de la demande: 01.04.1992
(73) Titulaire: ADIR ET COMPAGNIE, 92415 Courbevoie Cédex (FR)
(72) Inventeur: Taverne, Thierry, F-62222 Saint Martin Les Boulogne (FR); Lesieur, Isabelle, F-59147 Gondecourt (FR); Depreux, Patrick, F-59280 Armentieres (FR); Caignard, Daniel Henri, F-75015 Paris (FR); Guardiola, Béatrice, F-92200 Neuilly sur Seine (FR); Adam, Gérard, F-78600 Le Mesnil le Roi (FR); Renard, Pierre, F-78000 Versailles (FR)

(56) Documents cités:
- EP-A- 0 110 781
- EP-A- 0 171 702
- EP-A- 0 174 811
- EP-A- 0 281 309
- JOURNAL OF MEDICINAL CHEMISTRY, vol. 30, no. 7, Juillet 1987, WASHINGTON, US; pages 1166 - 1178; J. WEINSTOCK ET AL.: 'Synthesis and Evaluation of Non- Catechol D-1 and D-2 Dopamine Receptor Agon ists:Benzimidazol-2-one,Benzoxazol- 2-one,and the Highly Potent Benzothiazol-2-one 7-Ethylamines'

## Description

La présente invention concerne de nouvelles amines alkyl hétérocycliques, leur procédé de préparation et les compositions pharmaceutiques qui les contiennent.

De nombreuses amines alkyl hétérocycliques à motif benzoxazolinone, benzothiazolinone ou benzoxazinone ont déjà été décrites.

Le brevet européen EP 0 110781 décrit des (amino-2 éthyl)-6 benzoxazolinones en tant qu'hypnotiques et agents de traitement de l'insuffisance cardiaque. La demande de brevet européen EP 0 281309 décrit des composés de structure pipérazinoalkyl benzoxazolinoniques et benzothiazolinoniques intéressants en tant qu'antipsychotiques.

Le brevet français Fr 2035749 décrit des aminoalkyl benzoxazinones intéressantes pour le traitement des troubles du système nerveux central.

La publication "II farmaco" 89, 44 (1), 77-88 décrit des aryl pipérazinobutyl benzoxazolinones ainsi que leurs propriétés essentiellement analgésiques.

La demande de brevet EP 0 223 674 décrit des acyl-7 benzoxazinones et leurs dérivés comme possédant des propriétés antiarthéroscléreuses et normolipémiantes.

La demanderesse a présentement découvert de nouvelles amines alkyl hétérocycliques qui possèdent la propriété de se fixer avec une très haute affinité sur les récepteurs sérotoninergiques 5HT₁A. Cette affinité se double d'une excellente spécificité alors que les dérivés les plus proches de l'art antérieur étaient décrits comme des antagonistes dopaminergiques (Acta Ther 87, 13 (2), 125-139). Or l'affinité des dérivés de l'invention pour les récepteurs dopaminergiques est sensiblement plus faible que celle qu'ils manifestent pour les récepteurs sérotoninergiques.

La très haute affinité des dérivés de l'invention pour les récepteurs sérotoninergiques les rend utilisables dans le traitement des maladies du système sérotoninergique et plus particulièrement la dépression, le stress, l'anxiété et la schizophrénie, à des doses plus faibles que les dérivés de l'art antérieur. Cette particularité jointe à leur faible toxicité rend les composés de l'invention utilisables avec une sureté beaucoup plus grande que les composés de l'art antérieur ce qui est particulièrement intéressant compte tenu de la fragilité des populations auxquelles s'adresse ce type de traitement.

En outre certains dérivés de l'invention possèdent de bonnes propriétés analgésiques, hypnotiques, antihypertensives ou peuvent être utilisés dans la prévention de l'athérome.

Plus spécifiquement, la présente invention concerne les dérivés de formule générale (I) : 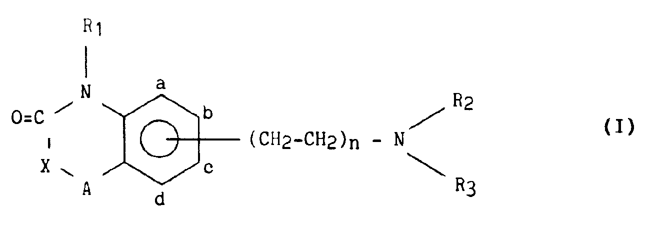 dans laquelle :
R₁ représente un atome d'hydrogène ou un groupement alkyle inférieur,
n représente 1 ou 2,
A représente un atome d'oxygène ou de soufre,
X représente un groupement CH₂ ou une liaison simple,
R₂ et R₃ forment ensemble avec l'atome d'azote qui les porte un système monocyclique à 6 sommets incluant un deuxieme hétéroatome choisi parmi azote ou oxygène le cas échéant substitué ou non sur l'éventuel atome d'azote par un ou plusieurs groupements alkyle inférieur, phényle, phénylalkyle inférieur, pyridyle, pyrimidinyle ou phényle substitué par un ou plusieurs groupements alkyle inférieur, trifluorométhyle, alkoxy inférieur ou atomes d'halogène ou phénylalkyle inférieur substitué sur le noyau phényle par un ou plusieurs groupements alkyle inférieur, trifluorométhyle, alkoxy inférieur ou atomes d'halogène ou pyridyle substitué par un ou plusieurs groupements alkyle inférieur, trifluorométhyle, alkoxy inférieur ou atomes d'halogène étant entendu que dans ce cas :
   - lorsque A représente un atome d'oxygène et que X est un groupement CH₂, le groupement (CH₂-CH₂)-NR₂R₃ ne peut se trouver en position b,
   - lorsque A représente un atome d'oxygène, que X est une liaison simple et que n = 1, le groupement (CH₂-CH₂)ₙ-NR₂R₃ ne peut se trouver en position c,

   étant entendu également que par "alkyle inférieur", on entend alkyle (C₁-C₆) linéaire ou ramifié,

leurs énantiomères, diastéréoisomères et épimères ainsi que leurs sels d'addition à un acide pharmaceutiquement acceptable ou une base pharmaceutiquement acceptable lorsque R₁ = H.

Parmi les acides pharmaceutiquement acceptables, on peut citer à titre non limitatif les acides chlorhydrique, sulfurique, tartrique, maléique, fumarique, oxalique, méthane sulfonique, camphorique, éthane sulfonique, citrique etc... Parmi les bases pharmaceutiquement acceptables, on peut citer à titre non limitatif les hydroxydes de sodium, potassium, calcium ainsi que les carbonates de sodium, potassium, calcium etc...

L'invention s'étend aussi au procédé de préparation des dérivés de formule générale (I) caractérisé en ce que l'on utilise comme matière première un dérivé de formule (II) : 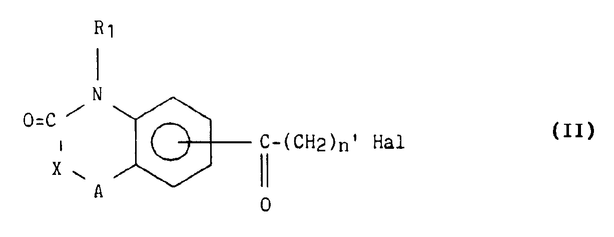 avec Hal représentant un atome d'halogène et R₁, A, X ayant la même définition que dans la formule (I) et n' représente 1 ou 3,
que l'on traite par un trialkylsilane en milieu acide pour conduire à un dérivé de formule (III) : 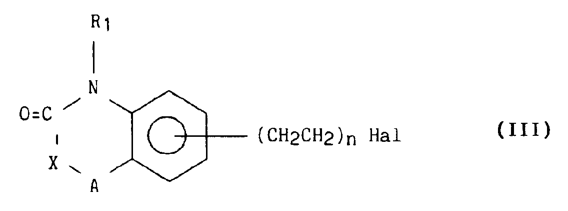 avec A, X, R₁, n et Hal tels que définis précédemment,
que l'on condense :
ou bien avec une amine de formule : 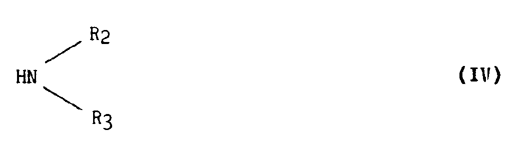 avec R₂ et R₃ ayant la même définition que précédemment, pour conduire à un dérivé de formule (1), 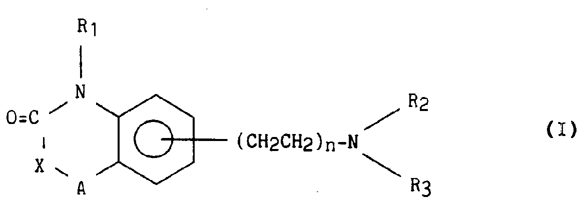 avec Rᵢ, X, A, n, R₂ et R₃ ayant la même définition que précédemment,
   dont on sépare le cas échéant les isomères que l'on purifie si nécessaire par chromatographie ou cristallisation, dérivé de formule (I) qui peut être, si on le désire, salifié par un acide ou une base pharmaceutiquement acceptable.

Les composés de formule (I) possèdent des propriétés pharmacologiques intéressantes.

Les essais de binding ont montré que les composés de l'invention se comportent comme de très puissants ligands des récepteurs 5-HT₁A. Cette affinité est accompagnée d'une très grande sélectivité vis à vis des autres récepteurs notamment D₂ et _{U2} contrairement à ce que l'on retrouve chez les dérivés de l'art antérieur.

Les composés de l'invention sont peu toxiques possèdent une bonne activité sur le test du conflit chez le pigeon, confirmant l'activité décelée par le binding. Certains d'entre eux possèdent par ailleurs une excellente activité analgésique d'autres une remarquable activité hypnotique, antihypertensive.

Les composés de l'invention trouvent donc leur application dans le traitement du stress, de l'anxiété, de la dépression, de la schizophrénie, des psychoses, démences, démences séniles, agressivité, agitation mais également, pour certains d'entre eux, dans les manifestations douloureuses sous toutes formes, les troubles du sommeil, l'hypertension artérielle, le glaucome.

La présente invention a également pour objet les compositions pharmaceutiques renfermant comme principe actif au moins un composé de formule générale (I) ou un de ses sels d'addition à un acide pharmaceutiquement acceptable ou le cas échéant à une base pharmaceutiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublingaux, les sachets, paquets, les gélules, glossettes, tablettes, suppositoires, crèmes, pommades, gels dermiques etc....

La posologie varie selon l'âge et le poids du patient, la nature et la sévérité de l'affection ainsi que la voie d'administration. Celle-ci peut être orale, nasale, rectale ou parentérale.

D'une manière générale, la posologie unitaire s'échelonne entre 0,05 et 30 mg pour les affections du comportement psychique et entre 1 mg et 500 mg pour le traitement de la douleur et de l'hypertension artérielle à savoir de une à trois prises par 24 heures.

Les exemples suivants illustrent l'invention et ne la limitent en aucune façon.

Les spectres de résonance magnétique nucléaire ¹ H ont été réalisés en utilisant le TMS (tétraméthylsilane) comme référence interne. Les déplacements chimiques sont exprimés en partie par million (p.p.m.). Les spectres infrarouge ont été effectués sous forme de pastille de bromure de potassium renfermant environ 1% du produit à analyser.

Les préparations ne font pas partie de l'invention mais sont utiles pour réaliser la synthèse des dérivés de l'invention.

### PREPARATION 1 :

### 6-BROMOACETYL BENZOTHIAZOLINONE

Dans une fiole à col rodé de 500 cm³ surmontée d'un réfrigérant, introduire 210 g (1,60 mole) de chlorure d'aluminium puis, goutte à goutte et sous agitation magnétique, 43 cm³ de diméthylformamide par l'intermédiaire d'une ampoule à brome. Ensuite, ajouter 30,2 g (0,2 mole) de benzothiazolinone, et pendant que le milieu réactionnel s'homogénéise, stabiliser la température à 70°C à l'aide d'un bain d'huile. Ensuite introduire peu à peu 19,8 cm³ (0,24 mole) du chlorure de l'acide bromoacétique. Après addition, laisser une heure sous agitation à la température de 70°C. Hydrolyser en versant le milieu réactionnel sur de la glace pilée, essorer le précipité obtenu, laver abondamment à l'eau et sécher. Recristalliser dans le dioxane.

Rendement : 65%

Point de fusion : 235°C avec décomposition

### PREPARATION 2 :

### 6-(2-BROMO ETHYL) BENZOTHIAZOLINONE

Dans une fiole à col rodé de 500 cm³ surmontée d'un réfrigérant et placée dans un bain d'huile, dissoudre 40,8 g (0,15 mole) de 6-bromoacétyl benzothiazolinone dans 90 cm³ d'acide trifluoroacétique sous agitation magnétique et en stabilisant la température à 60°C. Par l'intermédiaire d'une ampoule à brome, introduire goutte à goutte 52,7 cm³ (0,33 mole) de triéthylsilane. Après addition, arrêter le chauffage puis laisser 30 heures sous vive agitation. Hydrolyser en versant le milieu réactionnel dans l'eau glacée, essorer le précipité obtenu, laver à l'eau jusqu'à neutralité du filtrat puis à l'hexane. Sécher et recristalliser dans l'éthanol absolu.

Rendement : 80%

Point de fusion : 179-180°C

### PREPARATION 3 :

### 3-METHYL BENZOTHIAZOLINONE

Dans une fiole de 2 litres dissoudre 75,6 g (0,5 mole) de benzothiazolinone dans une solution contenant 20 g d'hydroxyde de sodium (0,5 mole) dans 800 cm³ d'eau environ. Filtrer la solution. Sous agitation magnétique, verser goutte à goutte 47,5 cm³ de sulfate de méthyle (0,5 mole) avec une ampoule à brome. Après addition, laisser 20 heures sous agitation à température ambiante. Alcaliniser le milieu avec un léger excès de soude, laisser agiter une heure. Essorer le précipité obtenu, laver à l'eau jusqu'à neutralité du filtrat. Sécher. Recristalliser dans le propanol.

Rendement : 88%

Point de fusion : 72-74°C

### PREPARATION 4 :

### 3-METHYL 6-BROMOACETYL BENZOTHIAZOLINONE

Dans une fiole à col rodé de 500 cm³ surmontée d'un réfrigérant, introduire 210 g (1,60 mole) de chlorure d'aluminium puis, goutte à goutte et sous agitation magnétique, 43 cm³ de diméthylformamide par l'intermédiaire d'une ampoule à brome. Ensuite ajouter 33 g (0,20 mole) de 3-méthyl benzothiazolinone, et pendant que le milieu réactionnel s'homogénéise, stabiliser la température à 70°C à l'aide d'un bain d'huile. Ensuite ajouter peu à peu 19,8 cm³ (0,24 mole) du chlorure de l'acide bromoacétique. Après addition, laisser une heure sous agitation à la température de 70°C. Hydrolyser en versant le milieu réactionnel sur de de la glace pilée, essorer le précipité obtenu, laver à l'eau jusqu'à neutralité du filtrat et sécher. Recristalliser dans l'alcool à 95°.

Rendement : 66%

Point de fusion : 164-165°C

### PREPARATION 5 :

### 3-METHYL 6-(2-BROMOETHYL) BENZOTHIAZOLINONE

Dans une fiole à col rodé de 500 cm³ surmontée d'un réfrigérant et placée dans un bain d'huile, dissoudre 42,9 g (0,15 mole) de 3-méthyl-6-bromoacétyl benzothiazolinone dans 77 cm³ d'acide trifluoroacétique sous agitation magnétique et en stabilisant la température à 60°C. Par l'intermédiaire d'une ampoule à brome, introduire goutte à goutte 52,7 cm³ (0,33 mole) de triéthylsilane. Après addition, arrêter le chauffage puis laisser 30 heures sous vive agitation. Hydrolyser en versant le milieu réactionnel dans l'eau glacée, essorer le précipité obtenu, laver à l'eau jusqu'à neutralité du filtrat puis à l'hexane. Sécher et recristalliser dans le cyclohexane.

Rendement : 86%

Point de fusion : 97-98°C

### PREPARATION 6 :

### 7-BROMOACETYL BENZOXAZINONE

Dissoudre 0,01 mole de 7-acétyl benzoxazinone décrit dans la demande EP 223 674 dans 100 cm³ de chlorure de méthylène. Par l'intermédiaire d'une ampoule à brome ajouter goutte à goutte et sous agitation 0,011 mole de brome et maintenir l'agitation pendant 13 heures. Filtrer, évaporer à sec et recristalliser le résidu.

### PREPARATION 7 :

### 6-(2-BROMOETHYL)3-OXO 2,3-DIHYDRO 1-4 BENZOXAZINE

Ce produit est obtenu avantageusement soit par hydrogénation catalytique de la 6-(bromoacétyl)3-oxo 2,3-dihydro 1,4-benzoxazine décrite dans la demande de brevet Français 2.035.749 en milieu acétique en présence de charbon palladié ou par action sur ce dérivé de trialkylsilane en milieu d'acide trifluorcacétique.

### PREPARATION 8 :

### 4-METHYL 7-(2-BROMOETHYL) 3-OXO 2,3-DIHYDRO 1,4-BENZOXAZINE

Dissoudre 0,01 mole de 4-méthyl 7-acétyl 3-oxo 2,3-dihydro 1,4-benzoxazine obtenue dans la demande de brevet européen 0223674 dans le chlorure méthylène. Par l'intermédiaire d'une ampoule à brome ajouter 0,012 mole de brome sous agitation.
Maintenir l'agitation deux heures puis abandonner le milieu réactionnel dans un bain d'huile à 40°C sous agitation pendant 2 heures. Filtrer. Evaporer le solvant. Recristalliser le résidu.
La 4-méthyl 7-bromoacétyl 3-oxo 2,3-dihydro benzoxazine est transformée en 4-méthyl-7-(2-bromoéthyl)3-oxo 2,3-dihydro 1,4-benzoxazine par hydrogénation catalytique en milieu acétique en présence de charbon palladié ou par action de trialkylsilane en milieu d'acide trifluoroacétique.

### PREPARATION 9 :

### 6-(2-PROPYLAMINOETHYL)3-OXO 2,3-DIHYDRO 1,4-BENZOXAZINE

Dans un ballon muni d'un agitateur mécanique et d'une garde à chlorure de calcium, ajouter 0,01 mole de n pro- pylamine et 0,015 mole de triéthylamine à une solution de 6-(2-bromoéthyl)3-oxo 2,3-dihydro 1,4 benzoxazine dans 40 cm³ de diméthylformamide. Chauffer à reflux 15 heures et essorer le précipité formé à chaud. Evaporer le résidu sous vide et recristalliser.

### PREPARATION 10 :

### 6-(4-BROMOBUTYL) BENZOTHIAZOLINONE

En procédant comme dans la préparation 2, mais en remplaçant la 6-bromoacétyl benzothiazolinone par la 6-(4-bromobutyryl) benzothiazolinone, on obtient le produit du titre.

### PREPARATION 11 :

### 3-METHYL 6-(4-BROMOBUTYL) BENZOTHIAZOLINONE

En procédant comme dans la préparation 5, mais en remplaçant la 6-bromoacétyl 3-méthyl benzothiazolinone par la 3-méthyl 6-(4-bromobutyryl) benzothiazolinone, on obtient le produit du titre.

### PREPARATION 12 :

### 7-(4-BROMOBUTYL) 4-METHYL 2,3-DIHYDRO 3-OXO BENZOXAZINE

En procédant comme dans la préparation 2, mais en remplaçant la 6-bromoacétyl benzothiazolinone par la 7-(4-bromobutyryl)4-méthyl 2,3-dihydro 3-oxo benzoxazine décrit dans la demande EP 0 223 674, on obtient le produit du titre.

### PREPARATION 13:

### 6-(4-BROMOBUTYL)BENZOXAZOLINONE

En procédant comme dans la préparation 2, mais en remplaçant la 6-bromoacétyl benzothiazolinone par la 6-(4-bromobutyryl) benzoxazolinone décrite dans la demande de brevet EP 0 281 309, on obtient le produit du titre.

### PREPARATION 14 :

### 3-METHYL 6-(4-BROMOBUTYL) BENZOTHIAZOLINONE

En procédant comme dans la préparation 13, mais en remplaçant la 6-(4-bromobutyryl)benzoxazolinone par la 3-méthyl 6-(4-bromobutyryl) benzoxazolinone, on obtient le produit du titre.

### EXEMPLE 28 :

### 3-METHYL 6-{2-[4-(2,3,4-TRIMETHOXY BENZYL) PIPERAZIN-1-YL]ETHYL} BENZOTHIAZOLINONE DICHLORHYDRATE

Dans une fiole à col rodé de 250 cm³ munie d'un réfrigérant à reflux, introduire 5,4 g (0,02 mole) de 3-méthyl 6-(2-bromo éthyl) benzothiazolinone préalablement dissoute dans 150 cm³ de dioxane puis 5,9 g (0,022 mole) de trimétazi- dine et 1 g de triéthylamine (0,01 mole). Chauffer à reflux sous agitation magnétique pendant 96 heures. Après refroidissement, essorer le mélange réactionnel, puis évaporer le filtrat au bain-marie sous vide. Reprendre le résidu par une solution d'HCI à 5%. Laver la phase aqueuse acide par l'acétate d'éthyle puis alcaliniser avec une solution de soude à 10%. Extraire avec l'éther, laver la phase organique avec l'eau distillée, puis la sécher sur chlorure de calcium. Filtrer et évaporer à sec au bain-marie sous vide. Reprendre dans l'éthanol absolu, barboter un courant d'HCI gazeux, essorer puis sécher. Recristalliser dans le méthanol.
Rendement : 52%
Point de fusion : 242 °C
Poids moléculaire : 530,52 g/mole
Composition centésimale : 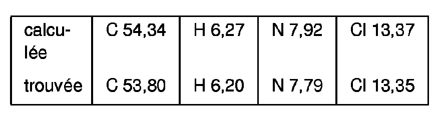
Spectrométrie dans l'infrarouae :
   3050-2800 cm⁻¹ : v (C-H)
   2700-2100 cm⁻¹ : v (NH+)
   1680 cn⁻¹ : v (C=O) -S-CO-N-
   1600-1580 cm⁻¹ : v (C=C) aromatique
Spectrométrie de Résonance Magnétique Nucléaire : Solvant : DMSO d₆
   δ = 3,14 à 3,61 ppm massif ; (12H) CH₂-CH₂-N et pipérazine
   δ = 3,39 ppm singulet ; (3H) ; NCH₃
   δ = 3,75-3,81-3,86 ppm singulets ; (3x3H) OCH₃
   δ = 4,19 ppm singulet ; (2H) N(CH₂)-triméthoxybenzyle
   δ = ₆,₇₅ à 7,56 ppm multiplet ; (5H) aromatiques
   δ = 11,75 ppm signal ; (2H) (NH+)

### EXEMPLES 29 A 31 :

### 3-METHYL 6- [2-(4-ARYL PIPERAZIN-1-YL)ETHYL]BENZOTHIAZOLINONE

Dans une fiole à col rodé de 250 cm³ munie d'un réfrigérant à reflux, introduire 5,4 g (0,02 mole) de 3-méthyl 6-(2-bromo éthyl) benzothiazolinone préalablement dissoute dans 150 cm³ de dioxane et 0,022 mole de 4-aryl pipérazine. Chauffer à reflux sous agitation magnétique pendant 96 heures.
Evaporer le mélange réactionnel au bain-marie sous vide, rincer le résidu avec une solution d'HCI à 5% puis avec de l'eau distillée, essorer, laver par l'acétate d'éthyle. Sécher et recristalliser.

### EXEMPLE 29 :

### 3-METHYL 6-{2-[4-(3-TRIFLUOROMETHYLPHENYL)PIPERAZIN-1-YL]ETHYL} BENZOTHIAZOLINONE MONOCHLORHYDRATE

Solvant de recristallisation : propanol ou éthanol
Rendement : 50%
Point de fusion : 236-237 °C
Poids moléculaire : 457,96 g/mole
Composition centésimale :

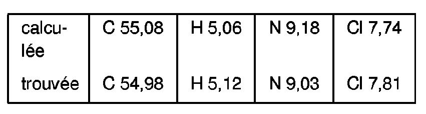

Spectrométrie dans l'infrarouge :
3100-2800 cm⁻¹ : v (C-H)
2700-2300 cm⁻¹ : v (NH+)
1680 cm⁻¹ : v (C=O) -S-CO-N-
1600-1580 cm⁻¹ : v (C=C) aromatique

Spectrométrie de Résonance Magnétique Nucléaire Solvant : DMSO d₆
δ = 3,21 à 3,84 ppm massif ; (12H) -CH₂
δ = 3,39 ppm singulet ; (3H) ; NCH₃
δ = ₇,₀₅ à 7,53 ppm multiplet ; (7H) aromatiques
δ = 11,32 ppm signal ; (1 H) (NH+)

### EXEMPLE 30 :

### 3-METHYL 6-{2-[4-(2-METHOXY PHENYL)PIPERAZIN-1-YL]ETHYL} BENZOTHIAZOLINONE MONOCHLORHYDRATE

Solvant de recristallisation : Méthanol
Rendement : 55%
Point de fusion : >250 °C
Poids moléculaire : 419,98 g/mole
Composition centésimale :

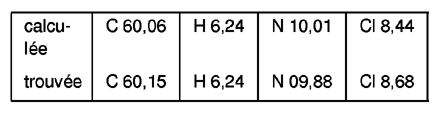

Spectrométrie dans l'infrarouge :
3000-2800 cm⁻¹ : v (C-H)
2750-2400 cm⁻¹ : v (NH₊)
1680 cm⁻¹ : v (C=O) -S-CO-NR-
1600-1580 cm⁻¹ : v (C=C) aromatique

Spectrométrie de Résonance Magnétique Nucléaire : Solvant : DMSO d₆
δ = 3,07 à 3,63 ppm massif ; (12H) -CH₂
δ ₌ 3,42 ppm singulet ; (3H) ; NCH₃
δ = 3,84 ppm singulet ; (3H) OCH₃
δ = 6,93 à 7,59 ppm multiplet ; (7H) aromatiques
δ = 11,31 ppm signal ; (1 H) (NH+)

### EXEMPLE 31 :

3-METHYL 6-{2-[4-(4-FLUOROPHENYL)PIPERAZIN-1-YL]ETHYL} BENZOTHIAZOLINONE DICHLORHYDRATE

Solvant de recristallisation : Méthanol
Rendement : 45%
Point de fusion : 234-235 °C
Poids moléculaire : 444,41 g/mole
Composition centésimale :

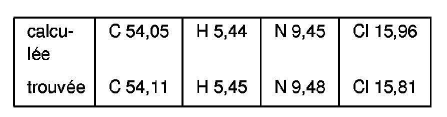

Spectrométrie dans l'infrarouge:
3100-2800 cm⁻¹ : v (C-H)
2700-2100 cm⁻¹ : v (NH+)
1680 cm⁻¹ : v (C=O) -O-CO-NR-
1600-1580 cm⁻¹ : v (C=C) aromatique

Spectrométrie de Résonance Magnétique Nucléaire : Solvant : DMSO d₆
δ = ₃,₂₆ à 3,82 ppm massif ; (12H) -CH₂
δ = 3,42 ppm singulet ; (3H) ; NCH₃
δ = 6,65 à 7,61 ppm multiplet ; (7H) aromatiques
δ = 11,82 ppm signal ; (2H) (NH+)

### EXEMPLE 32 :

### 6-{2-[4-(3-TRIFLUOROMETHYLPHENYL)PIPERAZIN-1-YL]ETHYL} BENZOTHIAZOLINONE

Dans une fiole rodée de 250 cm³ munie d'un réfrigérant à reflux, introduire 5,2 g (0,02 mole) de 6-(2-bromoéthyl) benzothiazolinone préalablement dissous dans 150 cm³ de dioxane puis 0,022 mole de 1-(3 trifluorométhyl phényl) pipérazine et 0,2 g d'iodure de potassium. Chauffer à reflux sous agitation magnétique pendant 96 heures. Evaporer le mélange réactionnel au bain-marie sous vide, triturer le précipité dans une solution d'HCI à 5%, essorer et laver à l'eau. Reprendre le précipité dans une solution aqueuse de Na₂C0₃ à 10% et extraire par l'acétate d'éthyle ; laver la phase organique par l'eau puis évaporer le solvant. Dissoudre le résidu dans un minimum d'éthanol et barboter un courant d'acide chlorhydrique gazeux, puis essorer le précipité. Reprendre le précipité dans de l'eau contenant 2 équivalents de K₂C0₃ et agiter pendant 1 à 2 heures. Essorer, laver le précipité avec l'eau distillée. Sécher et recristalliser dans le propanol.
Rendement : 45%
Point de fusion : 132 °C
Poids moléculaire : 407,47 g/mole
Composition centésimale :

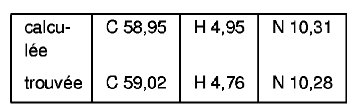

Spectrométrie dans l'infrarouge :
3160 cm⁻¹: v (N-H)
3100-2800 cm⁻¹ : v (C-H) -CH₂-
1680 cm⁻¹: v (C=O) -S-CO-NR-
1600-1580 cm⁻¹ : v (C=C) aromatique

Spectrométrie de Résonance Magnétique Nucléaire: Solvant : DMSO d₆
δ = 2,39 à 3,51 ppm massif ; (12H) -CH₂
δ = 6,96 à 7,57 ppm multiplet ; (7H) aromatiques
δ = 11,75 ppm signal ; (1H) -NH-

### EXEMPLES 86 A 89 :

En procédant comme dans les exemples 28 à 31, mais en remplaçant la 3-méthyl 6-(2-bromoéthyl) benzothiazolinone par la 4-méthyl 7-(4-bromo butyl)2,3-dihydro 3-oxo 1,4-benzoxazine, on obtient :

### EXEMPLE 86 :

### 4-METHYL 7-{4-[4-(2,3,4-TRIMETHOXYBENZYL) PIPERAZIN-1-YL)BUTYL} 2,3-DIHYDRO 3-OXO 1,4-BENZOXAZINE, DICHLORHYDRATE

### EXEMPLE 87 :

### 4-METHYL 7-{4-[4-(3-TRIFLUOROMETHYL PHENYL) PIPERAZIN-1-YL]BUTYL} 2,3-DIHYDRO 3-OXO 1,4-BENZOXAZINE, MONOCHLORHYDRATE

Point de fusion : 205°C

### EXEMPLE 88 :

### 4-METHYL 7-{4-[4-(2-METHOXY PHENYL)PIPERAZIN-1-YL]BUTYL} 2,3-DIHYDRO 3-OXO 1,4-BENZOXAZINE, BASE

Point de fusion : 89-90°C

### EXEMPLE 89 :

### 4-METHYL 7-{4-[4-(4-FLUOROPHENYL)PIPERAZIN-1-YL]BUTYL} 2,3-DIHYDRO 3-OXO 1,4-BENZOXAZINE, MONOCHLORHYDRATE

### EXEMPLES 127 A 130 :

En procédant comme dans les exemples 28 à 31, mais en remplaçant la 3-méthyl 6-(2-bromoéthyl) benzothiazolinone par la 3-méthyl 6-(4-bromobutyl) benzothiazolinone, on obtient respectivement :

### EXEMPLE 127:

### 3-METHYL 6-{4-[4-(2,3,4-TRIMETHOXYBENZYL) PIPERAZIN-1- YL] BUTYL} BENZOTHIAZOLINONE, DICHLORHYDRATE

### EXEMPLE 128:

### 3-METHYL 6-{4-[4-[3-TRIFLUOROMETHYLPHENYL) PIPERAZIN-1- YL] BUTYL} BENZOTHIAZOLINONE, MONOCHLORHYDRATE

Point de fusion : 182-184°C

### EXEMPLE 129 :

### 3-METHYL 6-{4-[4-(2-METHOXYPHENYL)PIPERAZIN-1-YL] BUTYL} BENZOTHIAZOLINONE, MONOCHLORHYDRATE

Point de fusion : 203-232°C

### EXEMPLE 130:

### 3-METHYL 6-{4-[4-(4-FLUOROPHENYL)PIPERAZIN-1-YL] BUTYL} BENZOTHIAZOLINONE

### EXEMPLE 131 :

### 6-{4-[4-(2-METHOXYPHENYL)PIPERAZIN-1-YL] BUTYL} BENZOTHIAZOLINONE

En procédant comme dans l'exemple 30, mais en remplaçant la 3-méthyl 6-(2-bromoéthyl)benzothiazolinone par la 6-(4-bromobutyl) benzothiazolinone sans acidifier en fin de manipulation, on obtient le produit du titre.
Solvant de recristallisation : toluène
Point de fusion : 125-127°C

### EXEMPLE 169:

### 6-{2-[4-(2-METHOXY PHENYL)PIPERAZIN-1 YL]ETHYL}BENZOTHIAZOLINONE}, CHLORHYDRATE

En procédant comme dans l'exemple 32 mais en remplaçant la 1-(3-trifluoroéthyl phényl)pipérazine par la 1-(2-méthoxy phényl)pipérazine, et en isolant le chlorhydrate en fin de manipulation (au lieu d'alcaliniser par le K2C03), on obtient le produit du titre. Recristalliser dans l'eau.

Point de fusion : supérieur à 260°C

### EXEMPLE 170 :

### 6-(2-MORPHOLINO ETHYL)BENZOTHIAZOLINONE, CHLORHYDRATE

En procédant comme dans l'exemple précédent mais en remplaçant la 1-(2-méthoxy phényl) pipérazine par la mor- pholine, on obtient le produit du titre.
Recristalliser dans l'alcool à 95°
Point de fusion : supérieur à 260°C

### EXEMPLE 171 :

### 3- METHYL 6-{4-MORPHOLINOBUTYL}BENZOTHIAZOLINONE, CHLORHYDRATE

En procédant comme dans l'exemple 129, mais en remplaçant la 1-(2-méthoxy phényl) pipérazine par la morpho- line, on obtient le produit du titre.
Solvant de recristallisation : toluène
Point de fusion : 171-173°C

### EXEMPLE 172 :

### 6-{4-[4-(3-TRIFLUOROMETHYLPHENYL)PIPERAZIN-1 YL]BUTYL}BENZOTHIAZOLINONE

En procédant comme dans l'exemple 131, mais en remplaçant la 1-(2-méthoxy phényl) pipérazine par la 1-(3-trifluorométhyl phényl) pipérazine, on obtient le produit du titre.
Solvant de recristallisation : alcool à 95°
Point de fusion : 129-130°C

### EXEMPLE 173 :

### 7-{2-[4-(3-TRIFLUOROMETHYLPHENYL)PIPERAZIN-1 YL]ETHYL}2,3-DIHYDRO 3-OXO 1,4-BENZOTHIAZINE

En procédant comme dans l'exemple 32, mais en remplaçant la 6-(2-bromoéthyl) benzothiazolinone par la 7-(2-bromoéthyl)2,3-dihydro 3-oxo 1,4-benzothiazine (obtenue à partir de la 7-acétyl 2,3-dihydro 3-oxo 1,4-benzothiazine décrite dans Ann. Chem. Rome, 1955, 45, 172) que l'on transforme en 7-bromoacétyl 2,3-dihydro 3-oxo 1,4-benzothiazine par action du brome en milieu chlorure de méthylène, cette matière première étant traitée par le triéthylsilane en milieu acide pour donner la 7-(2-bromoéthyl)2,3-dihydro 3-oxo 1,4-benzothiazine on obtient le produit du titre.

### EXEMPLE 174 :

### 3-METHYL 6-{2-[4-(3-PYRIDYL)PIPERAZIN-1 YL]ETHYL}BENZOTHIAZOLINONE, MONOCHLORHYDRATE

En procédant comme dans l'exemple 29, mais en remplaçant la 1-(3-trifluorométhyl phényl)pipérazine par la 1-(3-pyridyl)pipérazine, on obtient le produit du titre.

### EXEMPLE 175 :

### 3-METHYL 6-{2-[4-(2-PYRIMIDYL)PIPERAZIN-1-YL]ETHYL}BENZOTHIAZOLINONE, MONOCHLORHYDRATE

En procédant comme dans l'exemple 29, mais en remplaçant la 1-(3-trifluorométhyl phényl)pipérazine par la 1-(2- pyrimidyl) pipérazine, on obtient le produit du titre.

### EXEMPLE 176 :

### 4-METHYL 7-{4-[4-(2-PYRIDYL)PIPERAZIN-1-YL]BUTYL}2,3-DIHYDRO 3-OXO 1,4-BENZOXAZINE

En procédant comme dans l'exemple 87, mais en remplaçant la 1-(3-trifluorométhyl phényl) pipérazine par la 1-(2-pyridyl) pipérazine, on obtient le produit du titre.

### EXEMPLE 177 :

### 4-METHYL 7-{4-[4-(2-PYRIMIDYL)PIPERAZIN-1-YL]BUTYL}2,3-DIHYDRO 3-OXO 1,4-BENZOXAZINE

En procédant comme dans l'exemple 87, mais en remplaçant la 1-(3-trifluorométhyl phényl) pipérazine par la 1-(2- pyrimidyl) pipérazine, on obtient le produit du titre.

### EXEMPLE 178 :

### 8-{2-[4-(3-TRIFLUOROMETHYL PHENYL)PIPERAZIN-1-YL]ETHYL}2,3-DIHYDRO 3-OXO 1,4-BENZOTHIAZINE

En procédant comme dans l'exemple 173, mais en remplaçant la 7(2-bromoéthyl)2,3-dihydro 3-oxo 1,4-benzothiazine par la 8-(2-bromoéthyl)2,3-dihydro 3-oxo 1,4 benzothiazine (obtenue à partir de la 8-acétyl 2,3 dihydro 3-oxo 1,4-benzothiazine décrite dans Eur. J. Med. Chem. 89, 24 (5), 479-484), on obtient le produit du titre. En procédant de même que précédemment on obtient :
7-{2-[4-(4-FLUORO PHENYL)PIPERAZIN-1-YL]ETHYL}2,3-DIHYDRO 3-OXO 1,4-BENZOTHIAZINE
7-{2-[4-(2-METHOXY PHENYL)PIPERAZIN-1-YL]ETHYL}2,3-DIHYDRO 3-OXO 1,4-BENZOTHIAZINE
7-{2-[4-(PYRIMIDYL-2)PIPERAZIN-1-YL]ETHYL}2,3-DIHYDRO 3-OXO 1,4-BENZOTHIAZINE
7-{2-[4-(PYRIDYL-2)PIPERAZIN-1-YL]ETHYL}2,3-DIHYDRO 3-OXO 1,4-BENZOTHIAZINE

En utilisant les modes opératoires précédents mais à partir de la 6-(bromoéthyl)2,3-dihydro 3-oxo benzothiazine, les composés suivants sont obtenus :
6-{2-[4-(4-FLUORO PHENYL)-1-PIPERAZINYL]ETHYL]2,3-DIHYDRO 3-OXO 1,4-BENZOTHIAZINE
6-{2-[4-(2-METHOXY PHENYL)-1-PIPERAZINYL]ETHYL}2,3-DIHYDRO 3-OXO 1,4-BENZOTHIAZINE
6-{2-[4-(3-TRIFLUOROMETHYL PHENYL)-1-PIPERAZINYL]ETHYL}2,3-DIHYDRO 3-OXO 1,4-BENZOTHIAZINE
6-{2-[4-(PYRIMIDYL-2)-1-PIPERAZINYL]ETHYL}2,3-DIHYDRO 3-OXO 1,4-BENZOTHIAZINE
6-{2-[4-(PYRIDYL-2)-1-PIPERAZINYL]ETHYL}2,3-DIHYDRO 3-OXO 1,4-BENZOTHIAZINE

En utilisant les modes opératoires des exemples 26 à 31, mais en remplaçant la 3-méthyl 6-(2-bromoéthyl) benzothiazolinone par la 4-méthyl 7-(2-bromoéthyl) 2,3-dihydro 3-oxo benzoxazine, on obtient :
4-METHYL 7-{2-[4-(2,3,4-TRIMETHOXY BENZYL)PIPERAZIN-1-YL]ETHYL}2,3-DIHYDRO 3-OXO 1,4-BENZOXAZINE
4-METHYL 7-{2-[4-(3-TRIFLUOROMETHYL PHENYL)PIPERAZIN-1-YL]ETHYL}2,3-DIHYDRO 3-OXO 1,4-BENZOXAZINE
4-METHYL 7-{2-[4-(2-METHOXY PHENYL)PIPERAZIN-1-YL]ETHYL}2,3-DIHYDRO 3-OXO 1,4-BENZOXAZINE
4-METHYL 7-{2-[4-(4-FLUORO PHENYL)PIPERAZIN-1-YL]ETHYL}2,3-DIHYDRO 3-OXO 1,4-BENZOXAZINE
7-{2-[4-(3-TRIFLUOROMETHYL PHENYL)PIPERAZIN-1-YL]ETHYL}2,3-DIHYDRO 3-OXO 1,4-BENZOXAZINE
7-{2-[4-(2-METHOXY PHENYL)PIPERAZIN-1-YL]ETHYL}2,3-DIHYDRO 3-OXO 1,4-BENZOXAZINE
7-{2-[4-(4-FLUORO PHENYL)PIPERAZIN-1-YL]ETHYL}2,3-DIHYDRO 3-OXO 1,4-BENZOXAZINE
7-{4-[4-(3-TRIFLUOROMETHYL)PIPERAZIN-1-YL]BUTYL}2,3-DIHYDRO 3-OXO 1,4-BENZOXAZINE
7-{4-[4-(2-METHOXY PHENYL)PIPERAZIN-1-YL]BUTYL}2,3-DIHYDRO 3-OXO 1,4-BENZOXAZINE
7-{4-[4-(4-FLUORO PHENYL)PIPERAZIN-1-YL]BUTYL}2,3-DIHYDRO 3-OXO 1,4-BENZOXAZINE
4-METHYL 7-{2-[4-(3-TRIFLUOROMETHYL PHENYL)PIPERAZIN-1-YL]ETHYL}2,3-DIHYDRO 3-OXO 1,4-BENZOTHIAZINE
4-METHYL 7-{2-[4-(2-METHOXY PHENYL)PIPERAZIN-1-YL]ETHYL}2,3-DIHYDRO 3-OXO 1,4-BENZOTHIAZINE
4-METHYL 7-{2-[4-(4-FLUORO PHENYL)PIPERAZIN-1-YL]ETHYL}2,3-DIHYDRO 3-OXO 1,4-BENZOTHIAZINE
4-METHYL 7-{4-[4-(3-TRIFLUOROMETHYL PHENYL)PIPERAZIN-1-YL]BUTYL}2,3-DIHYDRO 3-OXO 1,4-BENZOTHIAZINE
4-METHYL 7-{4-[4-(2-METHOXY PHENYL)PIPERAZIN-1-YL]BUTYL}2,3-DIHYDRO 3-OXO 1,4-BENZOTHIAZINE
4-METHYL 7-{4-[4-(4-FLUORO PHENYL)PIPERAZIN-1-YL]BUTYL}2,3-DIHYDRO 3-OXO 1,4-BENZOTHIAZINE
7-{4-[4-(3-TRIFLUOROMETHYL PHENYL)PIPERAZIN-1-YL]BUTYL}2,3-DIHYDRO 3-OXO 1,4-BENZOTHIAZINE
7-{4-[4-(2-METHOXY PHENYL)PIPERAZIN-1-YL]BUTYL}2,3-DIHYDRO 3-OXO 1,4-BENZOTHIAZINE
7-{-[4-(4-FLUORO PHENYL)PIPERAZIN-1-YL]BUTYL}2,3-DIHYDRO 3-OXO 1,4-BENZOTHIAZINE
6-{2-[4-(4-FLUORO PHENYL)PIPERAZIN-1-YL]ETHYL}BENZOTHIAZOLINONE
6-{4-[4-(4-FLUORO PHENYL)PIPERAZIN-1-YL]BUTYL}BENZOTHIAZOLINONE
6-{2-[4-PHENYL PIPERAZIN-1-YL]ETHYL}BENZOTHIAZOLINONE et son homologue méthylé en 3
6-{4-[4-PHENYL PIPERAZIN-1-YL]BUTYL}BENZOTHIAZOLINONE et son homologue méthylé en 3
7-{2-[4-PHENYL PIPERAZIN-1-YL]ETHYL}2,3-DIHYDRO 3-OXO BENZOXAZINE et son homologue méthylé en 4
7-{4-[4-PHENYL PIPERAZIN-1-YL]BUTYL}2,3-DIHYDRO 3-OXO BENZOXAZINE et son homologue méthyle en 4
7-{2-[4-PHENYL PIPERAZIN-1-YL]ETHYL}2,3-DIHYDRO 3-OXO BENZOTHIAZINE et son homologue méthylé en 4
7-{4-[4-PHENYL PIPERAZIN-1-YL]BUTYL}2,3-DIHYDRO 3-OXO BENZOTHIAZINE et son homologue méthylé en 4

### ETUDE PHARMACOLOGIQUE DES DERIVES DE L'INVENTION :

### EXEMPLE 179 :

### TEST D'AFFINITE IN VITRO POUR LES RECEPTEURS 5-HT₁A, 5HT₂, D₂ ET α₂

Les tests d'affinité in vitro pour les récepteurs 5-HT₁A, 5HT₂, D₂ et a2 ont été réalisés selon des techniques classiques de binding.

Les résultats de ces études montrent que les composés de l'invention possèdent un K0,5 de l'ordre de 10⁻¹⁰M vis à vis des récepteurs 5-HT₁A. Cette très grande affinité est complétée par une très grande sélectivité. En effet le rapport des affinités 5-HT₁A/D₂ est égal à 100. Celui des affinités 5-HT₁A/α₂ est égal à 10^{4.} Celui des affinités 5HT₁A/5HT₂ est voisin de ₁₀³.

### EXEMPLE 180 :

### TOXICITE AIGUE

La toxicité aigue a été appréciée après administration orale à des lots de 8 souris (26 ±2 grammes) d'une dose de 650 mg.kg-¹. Les animaux ont été observés à intervalles réguliers au cours de la première journée et quotidiennement pendant les deux semaines suivant le traitement.
Il apparaît que la plupart des composés de l'invention sont totalement atoxiques. La plupart d'entre eux n'entraine aucun décès après administration à une dose de 650 mg.kg⁻¹ et on ne constate généralement pas de troubles après administration de cette dose.

### EXEMPLE 181 :

### ETUDE DE L'ACTIVITE ANXIOLYTIQUE - TEST DU CONFLIT CHEZ LE PIGEON

Six pigeons White Carneaux expérimentalement vierges sont entrainés à picorer une clé de plexiglas qui est tran- silluminée par des lumières rouges ou blanches. La clé réponse est montée sur la paroi frontale de la chambre expérimentale. Les pigeons sont amenés à 85% de leur poids normal avant le début de l'expérimentation laquelle est menée en utilisant la méthode des approximations successives (Frester 1953). Au départ, chaque picorage de la clé (illuminée avec une lumière rouge ou blanche) qui dépasse une force de 0,15N permet l'accès à un mélange de céréales par l'intermédiaire d'un distributeur automatique situé sous la clé. Après plusieurs jours les céréales ne sont plus délivrées qu'au trentième picorage sur la clé. Lorsque cette réponse au 30ème coup est obtenue, et qu'elle survient de façon régulière, permettant la délivrance de nourriture, la couleur de la lumière de la clé est alternée toutes les trois minutes (du blanc au rouge et vice-versa). La mesure du taux de réponse au 30ème coup reste en vigueur pendant chaque phase lumineuse.

Pendant cette phase et pour la durée de l'expérience une session quotidienne se compose de 5 cycles de 3 minutes de chaque séquence lumineuse, ces séquences étant séparées par une pause de 30 secondes pendant laquelle les clés lumineuses sont éteintes et les réponses n'ont pas de conséquence. Par conséquent, une séquence dure environ 35 à 40 minutes. Lorsque ces taux de réponses sont stables et identiques pour chaque couleur pendant une période de 5 jours (cela nécessite 3 à 4 semaines) chaque 30ème réponse dans l'une des phases colorées entraîne simultanément une libération de nourriture et un choc électrique bref (200 milli secondes) et modéré (1,3 mA) délivré par des electrodes placées au niveau des os pubiens. Le taux de réponses est réduit dans un premier temps puis retourne au niveau initial. L'administration des produits de l'invention est réalisée après l'obtention d'un taux de réponse stable sur une période de 5 jours. L'administration intramusculaire des produits de l'invention à une dose de 0,3 mg.kg-¹ entraîne une augmentation significative de réponses suivies ou non de chocs électriques prouvant l'activité anxiolytique de ces produits.

### EXEMPLE 182 :

### ETUDE DE L'ACTIVITE ANALGESIQUE

L'activité sur la douleur a été recherchée chez la souris (23-25 g) selon un protocole dérivé de la technique décrite par SIEGMUND (SIEGMUND E.A., R.A. CADMUS & GOLU, J. Pharm. Exp. Ther. 119, 1984,1954). Les souris, réparties par randomisation en lots de 12 animaux, ont reçu le traitement par voie orale (excipient pour les témoins) 1 heure avant l'injection intra-péritonéale d'une solution hydroalcoolique de phényl-p-benzoquinone (Sigma) à 0,02 %. Les étirements sont dénombrés entre la 5ème et 10ème minute après l'injection.

Le pourcentage d'activité obtenu a été évalué pour chaque dose (% de diminution du nombre d'étirements chez les traités par rapport aux témoins). Une ED₅₀, dose entraînant une activité de 50%, a été déterminée pour chaque produit.

Il est apparu que certains composés de l'invention possèdent une activité analgésique très intéressante. Ainsi, l'ED₅₀ du composé de l'exemple 31 est voisine de 5 mg.kg-¹.

### EXEMPLE 183 :

### ETUDE DE L'ACTIVITE HYPNOTIQUE

Des souris de sexe male, de souche OF1 IFFA-CREDO, d'un poids moyen de 22 +2 grammes reçoivent par intubation oesophagique, à raison de 0,25 ml pour 20 g de poids corporel d'une solution constituée de gomme arabique contenant le composé à étudier à savoir de 10 mg.kg-¹. On note le temps d'endormissement après avoir placé l'animal en decubitus dorsal et la durée du sommeil. Le pentobarbital est pris comme référence à une dose de 50 mg.kg-¹.

Il apparaît que certains composés de l'invention à une dose de 10 mg.kg-¹ ont une activité hypnotique supérieure à celle du pentobarbital à une dose de 50 mg.kg⁻¹ pris comme référence.

### EXEMPLE 184 :

### ETUDE DE L'ACTIVITE ANTIHYPERTENSIVE

L'expérimentation a été conduite sur des rats males spontanément hypertendus (souche OKAMOTO - élevage Charles River) de 23 à 24 semaines. Les produits de l'invention ont été administrés par voie orale dans un mélange eau - sirop de gomme 50/50) en traitement quotidien pendant deux jours à la dose quotidienne de 30 mg.kg-¹, sous un volume de 1 ml.kg⁻¹ par gavage, à l'aide d'une sonde oesophagienne.
Chaque dose a été administrée à un groupe de cinq rats. Un placebo (eau) 1 ml.kg-¹ a été administré en tant que témoin. La pression artérielle a été déterminée à la queue du rat selon la méthode de BYRON et WILSON (1938).

Pour ce faire, un brassard pneumatique en caoutchouc relié à un électrosphygnomanomètre NARCO type PE 300 est fixé au niveau de la queue de rats à 2 cm de la base de manière à comprimer l'artère caudale. Un capteur de pulsation type pneumatique permet d'ausculter l'artère à un centimètre en aval du brassard. La valeur de la pression artérielle systolique est celle pour laquelle on observe, au cours du jonglage du mamelon la réapparition des oscillations systolo-diastoliques.
Cinq mesures de pression sont effectuées pour chaque animal. Après élimination des deux valeurs extrêmes, la moyenne des trois valeurs restantes est calculée.
La moyenne des pressions artérielles systoliques par groupe de cinq rats est calculée avant tout traitement deux heures et 24 heures après le premier gavage.
Certains produits de l'invention ont des propriétés antihypertensives significatives.

### EXEMPLE 185 :

### COMPOSITIONS PHARMACEUTIQUES

Comprimés destinés au traitement des affections psychiques dosés à 5 mg de 3-{4-{N[2-(3-méthyl benzothiazolinone-6-yl)éthyl]amino}butyl}2,4-dioxo 3-aza spiro[4,5] décane.

### Formule de préparation pour 1000 comprimés

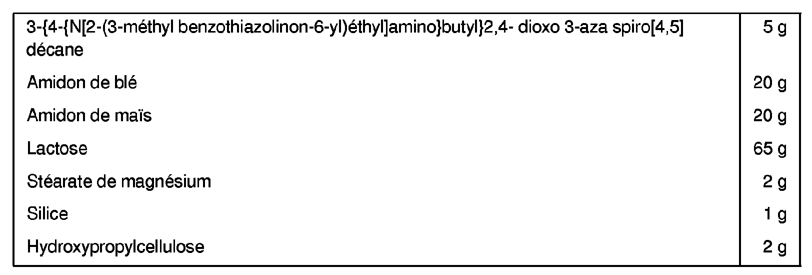

Comprimés destinés au traitement des affections psychiques dosés à 2,5 mg de 3-méthyl 6-{4-[4-(2-méthoxy phényl- pipérazin-1 yl]butyl} benzothiazolinone, chlorhydrate.

Formule de préparation pour 1000 comprimés

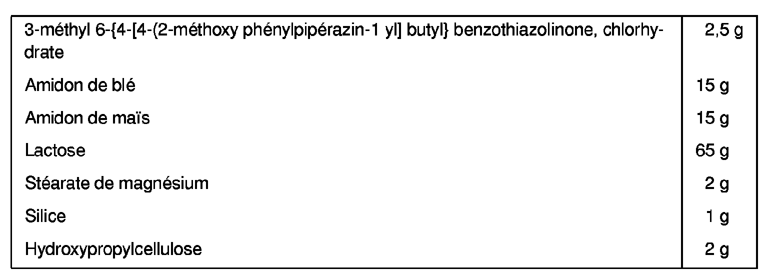

## Revendications (Revendications pour l'(les) Etat(s) contractant(s) suivant(s) : AT, BE, CH, DE, DK, FR, GB, IT, LI, LU, NL, SE)

1. Composés de formule générale (I) : 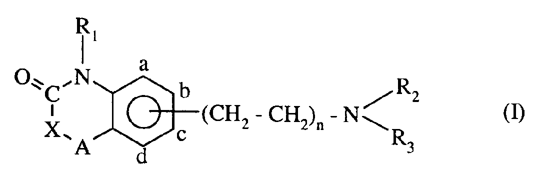 dans laquelle :
R₁ représente un atome d'Hydrogène ou un groupement alkyle inférieur,
n représente 1 ou 2,
A représente un atome d'oxygène ou de soufre,
X représente un groupement CH₂ ou une liaison simple,
R₂ et R₃ forment ensemble avec l'atome d'azote qui les porte un système hétérocyclique monocyclique à six sommets incluant un deuxième hétéroatome choisi parmi azote ou oxygène, le cas échéant substitué ou non sur l'éventuel atome d'azote par un groupement alkyle inférieur, phényle, phénylalkyle inférieur, pyridyle, pyrimidyle ou phényle substitué par un ou plusieurs groupements alkyle inférieur, trifluorométhyle, alkoxy inférieur ou atomes d'halogène ou phénylalkyle inférieur substitué sur le noyau phényle par un ou plusieurs groupements alkyle inférieur, trifluorométhyle, alkoxy inférieur ou atomes d'halogène ou pyridyle substitué par un ou plusieurs groupements alkyle inférieur, trifluorométhyle, alkoxy inférieur ou atomes d'halogène, étant entendu que :
- lorsque A représente un atome d'oxygène et que X est un groupement CH₂, le groupement (CH₂-CH₂)-NR₂R₃ ne peut se trouver en position b,
- lorsque A représente un atome d'oxygène, que X est une liaison simple et que n = 1, le groupement (CH₂-CH₂)ₙ-NR₂R₃ ne peut se trouver en position c,
étant entendu également que par "alkyle inférieur", on entend alkyle (C₁-C₆) linéaire ou ramifié,
leurs énantiomères, diastéréoisomères et épimères ainsi que leurs sels d'addition à un acide pharmaceutiquement acceptable ou une base pharmaceutiquement acceptable lorsque R₁ = H.

2. Composés de formule (I) selon la revendication 1 pour lesquels X représente une liaison simple, leurs énantiomères, épimères, diastéréoisomères ainsi que leur sels d'addition à un acide pharmaceutiquement acceptable ou une base pharmaceutiquement acceptable lorsque R₁ = H.

3. Composés de formule (I) selon la revendication 1 pour lesquels X représente un groupement CH₂, leurs énantiomères, épimères, diastéréoisomères ainsi que leurs sels d'addition à un acide pharmaceutiquement acceptable ou une base pharmaceutiquement acceptable lorsque R₁ = H.

4. Composés de formule (I) selon la revendication 1 pour lesquels A représente un atome d'oxygène et X une liaison simple, leurs énantiomères, épimères, diastéréoisomères ainsi que leurs sels d'addition à un acide pharmaceutiquement acceptable ou une base pharmaceutiquement acceptable lorsque R₁ = H.

5. Composé de formule (I) selon la revendication 1 pour lesquels A représente un atome de soufre, leurs énantiomères, épimères, diastéréoisomères ainsi que leurs sels d'addition à un acide pharmaceutiquement acceptable ou une base pharmaceutiquement acceptable lorsque R₁ = H.

6. Composés de formule (I) selon la revendication 1 pour lesquels A représente un atome d'oxygène et X représente un groupement CH₂, leurs énantiomères, épimères, diastéréoisomères ainsi que leurs sels d'addition à un acide pharmaceutiquement acceptable ou une base pharmaceutiquement acceptable lorsque R₁ = H.

7. Composés de formule (I) selon la revendication 1 pour lesquels A représente un atome de soufre et X représente une liaison simple, leurs énantiomères, épimères, diastéréoisomères ainsi que leurs sels d'addition à un acide pharmaceutiquement acceptable ou une base pharmaceutiquement acceptable lorsque R₁ = H.

8. Composé de formule (I) selon la revendication 1 pour lesquels le groupement (CH₂-CH₂)ₙNR₂R₃ est en position b, leurs énantiomères, épimères, diastéréoisomères ainsi que leurs sels d'addition à un acide pharmaceutiquement acceptable ou une base pharmaceutiquement acceptable lorsque R₁ = H.

9. Composé de formule (I) selon la revendication 1 pour lesquels le groupement (CH₂-CH₂)ₙNR₂R₃ est en position c, leurs énantiomères, épimères, diastéréoisomères ainsi que leurs sels d'addition à un acide pharmaceutiquement acceptable ou une base pharmaceutiquement acceptable lorsque R₁ = H.

10. Composé de formule (I) selon la revendication 1 pour lesquels le groupement (CH₂-CH₂)ₙNR₂R₃ est en position d, leurs énantiomères, épimères, diastéréoisomères ainsi que leurs sels d'addition à un acide pharmaceutiquement acceptable ou une base pharmaceutiquement acceptable lorsque R₁ = H.

11. Composés de formule (I) selon la revendication 1 pour lesquels X représente une liaison simple, A représente un atome de soufre et R₂ et R₃ forment avec l'atome d'azote qui les porte une pipérazine éventuellement substituée au niveau de l'azote pipérazinique numéroté 4 par un groupement phényle, phénylalkyle inférieur ou pyridyle, les groupements phényle, phénylalkyle ou pyridyle pouvant eux-mêmes être éventuellement substitués sur le noyau aromatique par un ou plusieurs groupements alkyle inférieur, trifluorométhyle, alkoxy inférieur ou atopmes d'halogène, leurs énantiomères, épimères, diastéréoisomères ainsi que leurs sels d'addition à un acide pharmaceutiquement acceptable ou une base pharmaceutiquement acceptable lorsque R₁ = H.

12. Composés de formule (I) selon la revendication 1 qui sont la 6-{2-[4-(3-trifluorométhylphényl)pipérazin-1-yl]éthyl]benzothiazolinone, son homologue méthylé en 3 ainsi que leurs sels d'addition à un acide pharmaceutiquement acceptable.

13. Composés de formule (I) selon la revendication 1 qui sont la 6-{2-[4-(2-méthoxyphényl)pipérazin-1-yl]éthyl}benzothiazolinone, son homologue méthylé en 3 ainsi que leurs sels d'addition à un acide pharmaceutiquement acceptable.

14. Composés de formule (I) selon la revendication 1 qui sont la 6-{2-[4-(4-fluorophényl) pipérazin-1-yl]éthyl}benzothiazolinone, son homologue méthylé en 3 ainsi que leurs sels d'addition à un acide pharmaceutiquement acceptable.

15. Composé selon la revendication 1 qui sont la 6-{4-[4-(2-méthoxyphényl)pipérazin-1-yl]butyl}benzothiazolinone, son homologue méthylé en 3 ainsi que leurs sels d'addition à un acide pharmaceutiquement acceptable.

16. Composés selon la revendication 1 qui sont les 7-{4-[4-arylpipérazin-1-yl]butyl}-2,3-dihydro-3-oxo-1,4-benzoxazine, leurs homologues méthylés en 4, aryl représentant un groupement phényle, 2-méthoxyphényle, 3-trifluorométhylphényle, ou 4-fluorophényle ainsi que leurs sels d'addition à un acide ou une base pharmaceutiquement acceptable.

17. Composés selon la revendication 1 qui les 7-{z-[4-aryl pipérazin-1-yl]alkyl}-2,3-dihydro-3-oxo-1,4-benzothiazine, leurs dérivés méthylés en 4, aryl représentant un groupement phényle, 4-fluorophényle, 2-méthoxyphényle ou 3-trifluorométhylphényle, alkyle signifie éthyle et dans ce cas z = 2 ou butyle et dans ce cas z = 4 ainsi que leurs sels d'addition à un acide ou une base pharmaceutiquement acceptable.

18. Composés de formule (I) selon la revendication 1 qui sont la 6-{2-[4-(2-pyrimidyl)-1-pipérazinyl)éthyl]benzothiazolinone, son homologue méthyle en 3 ainsi que leurs sels d'addition à un acide ou une base pharmaceutiquement acceptable.

19. Composés de formule (I) selon la revendication 1 qui sont la 6-{4-[4-(4-trifluorométhylphényl)-1-pipérazi- nyl)butyl]benzothiazolinone, son homologue méthylé en 3 ainsi que leurs sels d'addition à un acide ou une base pharmaceutiquement acceptable.

20. Composés de formule (I) selon la revendication 1 qui sont la 6-{4-[4-(4-fluorophényl)-1-pipérazinyl)butyl]benzothiazolinone, son homologue méthylé en 3 ainsi que leurs sels d'addition à un acide ou une base pharmaceutiquement acceptable.

21. Composés de formule (I) selon la revendication 1 qui sont des 7-{2-(4-aryl-1-pipérazinyl)éthyl]-2,3-dihydro-3-oxo- benzoxazines, leurs dérivés méthylés en 4, aryl signifiant un groupement phényle, 2-méthoxyphényle ou 4-fluorophényle ou 3-trifluorométhylphényle ainsi que leurs sels d'addition à un acide ou une base pharmaceutiquement acceptable.

22. Composés de formule (I) selon la revendication 1 qui sont la 6-{z-[4-phényl-1-pipérazinyl]alkyl}benzothiazolinone, ou alkyl représente un groupement éthyle et z = 2 ou butyle et z = 4, leurs homologues méthylés en 3 ainsi que leurs sels d'addition à un acide ou une base pharmaceutiquement acceptable.

23. Procédé de préparation de composés de formule (I) selon la revendication 1 caractérisé en ce que l'on utilise comme matière première un dérivé de formule (II) : 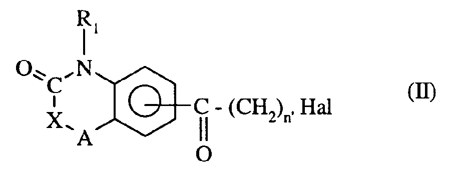 avec Hal représentant un atome d'halogène et R₁, A, X ayant la même définition que dans la formule (I) et n' représente 1 ou 3,
que l'on traite par un trialkysilane en milieu acide pour conduire à un dérivé de formule (III) : 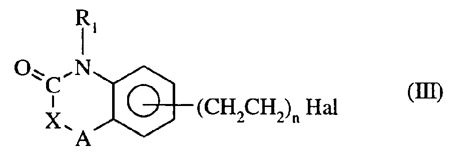 avec A, X, R₁, n et Hal tels que définis précédemment, que l'on condense ou bien avec une amine de formule (IV) : 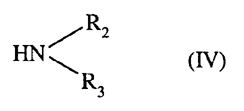 avec R₂ et R₃ ayant la même définition que précédemment, pour conduire à un dérivé de formule (I), 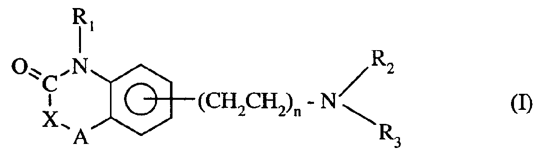 avec R₁, X, A, n, R₂ et R₃ ayant la même définition que précédemment,
dont on sépare le cas échéant les isomères que l'on purifie si nécessaire par chromatographie ou cristallisation, et qui peut être, si on le désire, salifié par un acide ou une base pharmaceutiquement acceptable.

24. Composition pharmaceutique contenant comme principe actif au moins un composé selon l'une des revendications 1 à 22 en combinaison avec un ou plusieurs excipients ou véhicules inertes, non toxiques, pharmaceutiquement acceptables.

25. Composition pharmaceutique selon la revendication 24 contenant au moins un principe actif selon l'une des revendications 1 à 22, utilisable en tant que psychotrope, analgésique ou antihypertenseur.

## Revendications (Revendications pour l'(les) Etat(s) contractant(s) suivant(s) : ES, GR)

1. Procédé de préparation de composés de formule générale (I) : 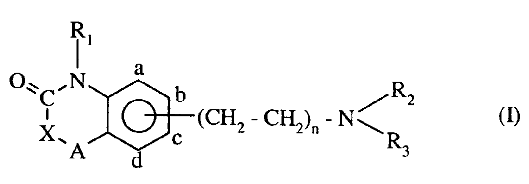 dans laquelle :
R₁ représente un atome d'hydrogène ou un groupement alkyle inférieur,
n représente 1 ou 2,
A représente un atome d'oxygène ou de soufre,
X représente un groupement CH₂ ou une liaison simple,
R₂ et R₃ forment ensemble avec l'atome d'azote qui les porte un système hétérocyclique monocyclique à six sommets incluant un deuxième hétéroatome choisi parmi azote ou oxygène, le cas échéant substitué ou non sur l'éventuel atome d'azote par un groupement alkyle inférieur, phényle, phénylalkyle inférieur, pyridyle, pyrimidyle ou phényle substitué par un ou plusieurs groupements alkyle inférieur, trifluorométhyle, alkoxy inférieur ou atomes d'halogène ou phénylalkyle inférieur substitué sur le noyau phényle par un ou plusieurs groupements alkyle inférieur, trifluorométhyle, alkoxy inférieur ou atomes d'halogène ou pyridyle substitué par un ou plusieurs groupements alkyle inférieur, trifluorométhyle, alkoxy inférieur ou atomes d'halogène,
étant entendu que :
- lorsque A représente un atome d'oxygène et que X est un groupement CH₂, le groupement (CH₂-CH₂)-NR₂R₃ ne peut se trouver en position b,
- lorsque A représente un atome d'oxygène, que X est une liaison simple et que n = 1, le groupement (CH₂-CH₂)ₙ-NR₂R₃ ne peut se trouver en position c,
étant entendu également que par "alkyle inférieur", on entend alkyle (C₁-C₆) linéaire ou ramifié,
leurs énantiomères, diastéréoisomères et épimères ainsi que leurs sels d'addition à un acide pharmaceutiquement acceptable ou une base pharmaceutiquement acceptable lorsque R₁ = H, caractérisé en ce que l'on utilise comme matière première un dérivé de formule (ll) : 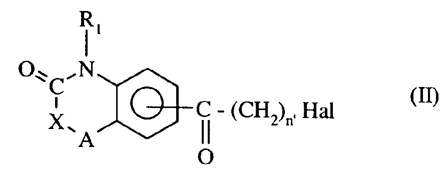 avec Hal représentant un atome d'halogène et R₁, A, X ayant la même définition que dans la formule (I) et n' représente 1 ou 3,
que l'on traite par un trialkysilane en milieu acide pour conduire à un dérivé de formule (lll) : 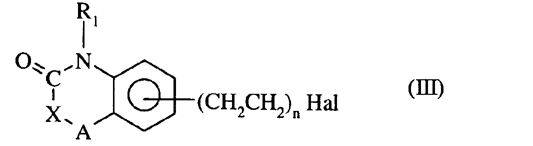 avec A, X, R₁, n et Hal tels que définis précédemment,
que l'on condense ou bien avec une amine de formule (IV) : 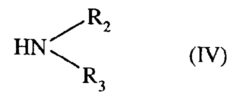 avec R₂ et R₃ ayant la même définition que précédemment, pour conduire à un dérivé de formule (1), 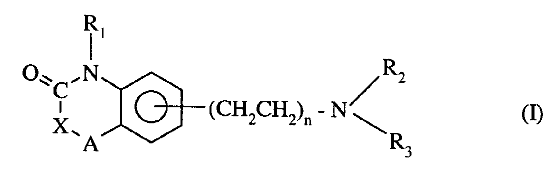 avec R₁, X, A, n, R₂ et R₃ ayant la même définition que précédemment,
dont on sépare le cas échéant les isomères que l'on purifie si nécessaire par chromatographie ou cristallisation, et qui peut être, si on le désire, salifié par un acide ou une base pharmaceutiquement acceptable.

2. Procédé de préparation selon la revendication 1 des composés de formule (I) pour lesquels X représente une liaison simple, leurs énantiomères, épimères, diastéréoisomères ainsi que leur sels d'addition à un acide pharmaceutiquement acceptable ou une base pharmaceutiquement acceptable lorsque R₁ = H.

3. Procédé de préparation selon la revendication 1 des composés de formule (I) pour lesquels X représente un groupement CH₂, leurs énantiomères, épimères, diastéréoisomères ainsi que leurs sels d'addition à un acide pharmaceutiquement acceptable ou une base pharmaceutiquement acceptable lorsque R₁ = H.

4. Procédé de préparation selon la revendication 1 des composés de formule (I) pour lesquels A représente un atome d'oxygène et X une liaison simple, leurs énantiomères, épimères, diastéréoisomères ainsi que leurs sels d'addition à un acide pharmaceutiquement acceptable ou une base pharmaceutiquement acceptable lorsque R₁ = H.

5. Procédé de préparation selon la revendication 1 des composés de formule (I) pour lesquels A représente un atome de soufre, leurs énantiomères, épimères, diastéréoisomères ainsi que leurs sels d'addition à un acide pharmaceutiquement acceptable ou une base pharmaceutiquement acceptable lorsque R₁ = H.

6. Procédé de préparation selon la revendication 1 des composés de formule (I) pour lesquels A représente un atome d'oxygène et X représente un groupement CH₂, leurs énantiomères, épimères, diastéréoisomères ainsi que leurs sels d'addition à un acide pharmaceutiquement acceptable ou une base pharmaceutiquement acceptable lorsque R₁ = H.

7. Procédé de préparation selon la revendication 1 des composés de formule (I) pour lesquels A représente un atome de soufre et X représente une liaison simple, leurs énantiomères, épimères, diastéréoisomères ainsi que leurs sels d'addition à un acide pharmaceutiquement acceptable ou une base pharmaceutiquement acceptable lorsque R₁ = H.

8. Procédé de préparation selon la revendication 1 des composés de formule (I) pour lesquels le groupement (CH₂-CH₂)ₙNR₂R₃ est en position b, leurs énantiomères, épimères, diastéréoisomères ainsi que leurs sels d'addition à un acide pharmaceutiquement acceptable ou une base pharmaceutiquement acceptable lorsque R₁ = H.

9. Procédé de préparation selon la revendication 1 des composés de formule (I) pour lesquels le groupement (CH₂-CH₂)ₙNR₂R₃ est en position c, leurs énantiomères, épimères, diastéréoisomères ainsi que leurs sels d'addition à un acide pharmaceutiquement acceptable ou une base pharmaceutiquement acceptable lorsque R₁ = H.

10. Procédé de préparation selon la revendication 1 des composés de formule (I) pour lesquels le groupement (CH₂-CH₂)ₙNR₂R₃ est en position d, leurs énantiomères, épimères, diastéréoisomères ainsi que leurs sels d'addition à un acide pharmaceutiquement acceptable ou une base pharmaceutiquement acceptable lorsque R₁ = H.

11. Procédé de préparation selon la revendication 1 des composés de formule (I) pour lesquels X représente une liaison simple, A représente un atome de soufre et R₂ et R₃ forment avec l'atome d'azote qui les porte une pipérazine éventuellement substituée au niveau de l'azote pipérazinique numéroté 4 par un groupement phényle, phénylalkyle inférieur ou pyridyle, les groupements phényle, phénylalkyle ou pyridyle pouvant eux-mêmes être éventuellement substitués sur le noyau aromatique par un ou plusieurs groupements alkyle inférieur, trifluorométhyle, alkoxy inférieur ou atopmes d'halogène, leurs énantiomères, épimères, diastéréoisomères ainsi que leurs sels d'addition à un acide pharmaceutiquement acceptable ou une base pharmaceutiquement acceptable lorsque R₁ = H.

12. Procédé de préparation selon la revendication 1 des composés de formule (I) qui sont la 6-{2-[4-(3-trifluorométhyl- phényl)pipérazin-1-yl]éthyl}benzothiazolinone, son homologue méthylé en 3 ainsi que leurs sels d'addition à un acide pharmaceutiquement acceptable.

13. Procédé de préparation selon la revendication 1 des composés de formule (I) qui sont la 6-{2-[4-(2-méthoxy-phényl)pipérazin-1-yl]éthyl}benzothiazolinone, son homologue méthylé en 3 ainsi que leurs sels d'addition à un acide pharmaceutiquement acceptable.

14. Procédé de préparation selon la revendication 1 des composés de formule (I) qui sont la 6-{2-[4-(4-fluorophényl) pipérazin-1-yl]éthyl}benzothiazolinone, son homologue méthylé en 3 ainsi que leurs sels d'addition à un acide pharmaceutiquement acceptable.

15. Procédé de préparation selon la revendication 1 des composés de formule (l) qui sont la 6-{4-[4-(2-méthoxyphé- nyl)pipérazin-1-yl]butyl}benzothiazolinone, son homologue méthylé en 3 ainsi que leurs sels d'addition à un acide pharmaceutiquement acceptable.

16. Procédé de préparation selon la revendication 1 des composés de formule (l) qui sont les 7-{4-[4-arylpipérazin-1-yl]butyl}2,3tühydro-3-oxo-1,4-benzoxazine, leurs homologues méthylés en 4, aryl représentant un groupement phényle, 2-méthoxyphényle, 3-trifluorométhylphényle, ou 4-fluorophényle ainsi que leurs sels d'addition à un acide ou une base pharmaceutiquement acceptable.

17. Procédé de préparation selon la revendication 1 des composés de formule (I) qui les 7-{z-[4-aryl pipérazin-1-yl]alkyl}2,3-dihydro-3-oxo-1,4-benzothiazine, leurs dérivés méthylés en 4, aryl représentant un groupement phényle, 4-fluorophényle, 2-méthoxyphényle ou 3-trifluorométhylphényle, alkyle signifie éthyle et dans ce cas z = 2 ou butyle et dans ce cas z = 4 ainsi que leurs sels d'addition à un acide ou une base pharmaceutiquement acceptable.

18. Procédé de préparation selon la revendication 1 des composés de formule (I) qui sont la 6-{2-[4-(2-pyrimidyl)-1-pipérazinyl)éthyl]benzothiazolinone, son homologue méthyle en 3 ainsi que leurs sels d'addition à un acide ou une base pharmaceutiquement acceptable.

19. Procédé de préparation selon la revendication 1 des composés de formule (I) qui sont la 6-{4-[4-(4-trifluorométhyl- phényl)-1-pipérazinyl)butyl]benzothiazolinone, son homologue méthylé en 3 ainsi que leurs sels d'addition à un acide ou une base pharmaceutiquement acceptable.

20. Procédé de préparation selon la revendication 1 des composés de formule (I) qui sont la 6-{4-[4-(4-fluorophényl)-1-pipérazinyl)butyl]benzothiazolinone, son homologue méthylé en 3 ainsi que leurs sels d'addition à un acide ou une base pharmaceutiquement acceptable.

21. Procédé de préparation selon la revendication 1 des composés de formule (I) qui sont les 7-{2-(4-aryl-1-pipérazi- nyl)éthyl]-2,3-dihydro-3-oxo-benzoxazines, leurs dérivés méthylés en 4, aryl signifiant un groupement phényle, 2-méthoxyphényle ou 4-fluorophényle ou 3-trifluorométhylphényle ainsi que leurs sels d'addition à un acide ou une base pharmaceutiquement acceptable.

22. Procédé de préparation selon la revendication 1 des composés de formule (I) qui sont la 6-{z-[4-phényl-1-pipérazi- nyl]alkyl}benzothiazolinone, où alkyl représente un groupement éthyle et z = 2 ou butyle et z = 4, leurs homologues méthylés en 3 ainsi que leurs sels d'addition à un acide ou une base pharmaceutiquement acceptable.

23. Procédé de préparation de compositions pharmaceutiques contenant comme principe actif au moins un composé obtenu selon l'une des revendications 1 à 22 en combinaison avec un ou plusieurs excipients ou véhicules inertes, non toxiques, pharmaceutiquement acceptables.

24. Procédé de préparation de compositions pharmaceutiques selon la revendication 24 contenant au moins un principe actif obtenu selon l'une des revendications 1 à 22, utilisable en tant que psychotrope, analgésique, antihypertenseur ou comme normolipémiant.

## Claims (Claims for the following Contracting State(s) : AT, BE, CH, DE, DK, FR, GB, IT, LI, LU, NL, SE)

1. Compounds of the general formula (I): 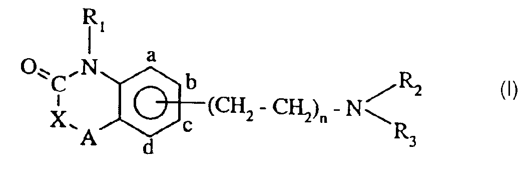 in which:
R₁ represents a hydrogen atom or a lower alkyl group,
n represents 1 or 2,
A represents an oxygen atom or a sulphur atom,
X represents a group CH₂ or a single bond, and
R₂ and R₃ form together with the nitrogen atom carrying them a monocyclic heterocyclic system having six ring members including a second hetero atom selected from nitrogen and oxygen, which heterocyclic system, where appropriate, is unsubstituted or substituted on the optional nitrogen atom by a lower alkyl, phenyl, phenyl-lower alkyl, pyridyl or pyrimidyl group; or by a phenyl group substituted by one or more groups lower alkyl, trifluoromethyl, lower alkoxy, or halogen atoms; or a phenyl-lower alkyl group substituted on the phenyl ring by one or more groups lower alkyl, trifluoromethyl, lower alkoxy, or halogen atoms; or by a pyridyl group substituted by one or more groups lower alkyl, trifluoromethyl, lower alkoxy, or halogen atoms;
wherein:
- when A represents an oxygen atom and X is a group CH₂, the group (CH₂-CH₂)ₙ-NR₂R₃ may not be in position b, and
when A represents an oxygen atom, X is a single bond and n is 1, the group (CH₂-CH₂)ₙ-NR₂R₃ may not be in position c,
and wherein "lower alkyl" is to be understood as meaning linear or branched (C₁-C_{G})alkyl,
their enantiomers, diastereoisomers and epimers, and addition salts thereof with a pharmaceutically acceptable acid or a pharmaceutically acceptable base when R₁ is hydrogen.

2. Compounds of formula (I) according to claim 1 in which X represents a single bond, their enantiomers, epimers and diastereoisomers, and addition salts thereof with a pharmaceutically acceptable acid or a pharmaceutically acceptable base when R₁ is hydrogen.

3. Compounds of formula (I) according to claim 1 in which X represents a group CH₂, their enantiomers, epimers and diastereoisomers, and addition salts thereof with a pharmaceutically acceptable acid or a pharmaceutically acceptable base when R₁ is hydrogen.

4. Compounds of formula (I) according to claim 1 in which A represents an oxygen atom and X represents a single bond, their enantiomers, epimers and diastereoisomers, and addition salts thereof with a pharmaceutically acceptable acid or a pharmaceutically acceptable base when R₁ is hydrogen.

5. Compounds of formula (I) according to claim 1 in which A represents a sulphur atom, their enantiomers, epimers and diastereoisomers, and addition salts thereof with a pharmaceutically acceptable acid or a pharmaceutically acceptable base when R₁ is hydrogen.

6. Compounds of formula (I) according to claim 1 in which A represents an oxygen atom and X represents a group CH₂, their enantiomers, epimers and diastereoisomers, and addition salts thereof with a pharmaceutically acceptable acid or a pharmaceutically acceptable base when R₁ is hydrogen.

7. Compounds of formula (I) according to claim 1 in which A represents a sulphur atom and X represents a single bond, their enantiomers, epimers and diastereoisomers, and addition salts thereof with a pharmaceutically acceptable acid or a pharmaceutically acceptable base when R₁ is hydrogen.

8. Compounds of formula (I) according to claim 1 in which the group (CH₂-CH₂),,NR₂R₃ is in position b, their enantiomers, epimers and diastereoisomers, and addition salts thereof with a pharmaceutically acceptable acid or a pharmaceutically acceptable base when R₁ is hydrogen.

9. Compounds of formula (I) according to claim 1 in which the group (CH₂-CH₂)ₙNR₂R₃ is in position c, their enantiomers, epimers and diastereoisomers, and addition salts thereof with a pharmaceutically acceptable acid or a pharmaceutically acceptable base when R₁ is hydrogen.

10. Compounds of formula (I) according to claim 1 in which the group (CH₂-CH₂)ₙNR₂R₃ is in position d, their enantiomers, epimers and diastereoisomers, and addition salts thereof with a pharmaceutically acceptable acid or a pharmaceutically acceptable base when R₁ is hydrogen.

11. Compounds of formula (I) according to claim 1 in which X represents a single bond, A represents a sulphur atom and R₂ and R₃ form with the nitrogen atom carrying them a piperazine that is optionally substituted on the piperazine nitrogen numbered 4 by a phenyl, phenyl-lower alkyl or pyridyl group, the phenyl, phenylalkyl and pyridyl groups themselves being optionally substituted on the aromatic ring by one or more groups lower alkyl, trifluoromethyl, lower alkoxy, or halogen atoms, their enantiomers, epimers and diastereoisomers, and addition salts thereof with a pharmaceutically acceptable acid or a pharmaceutically acceptable base when R₁ is hydrogen.

12. Compounds of formula (I) according to claim 1 which are 6-{2-[4-(3-trifluoromethylphenyl)piperazin-1-yl]ethyl}benzothiazolinone, its 3-methyl homologue, and addition salts thereof with a pharmaceutically acceptable acid.

13. Compounds of formula (I) according to claim 1 which are 6-{2-[4-(2-methoxyphenyl)piperazin-1-yl]ethyl}benzothiazolinone, its 3-methyl homologue, and addition salts thereof with a pharmaceutically acceptable acid.

14. Compounds of formula (I) according to claim 1 which are 6-{2-[4-(4-fluorophenyl)piperazin-1-yl]ethyl}benzothiazolinone, its 3-methyl homologue, and addition salts thereof with a pharmaceutically acceptable acid.

15. Compounds according to claim 1 which are 6-{4-[4-(2-methoxyphenyl)piperazin-1 -yl]butyl}benzothiazolinone, its 3-methyl homologue, and addition salts thereof with a pharmaceutically acceptable acid.

16. Compounds according to claim 1 which are 7-{4-[4-arylpiperazin-1-yl]butyl}-2,3-dihydro-3-oxo-1,4-benzoxazine, their 4-methyl homologues, with aryl representing a phenyl, 2-methoxyphenyl, 3-trifluoromethylphenyl or 4-fluorophenyl group, and addition salts thereof with a pharmaceutically acceptable acid or base.

17. Compounds according to claim 1 which are 7-{z-[4-arylpiperazin-1-yl]alkyl}-2,3-dihydro-3-oxo-1,4-benzothiazine, their 4-methyl derivatives, with aryl representing a phenyl, 4-fluorophenyl, 2-methoxyphenyl or 3-trifluoromethylphenyl group, and alkyl representing ethyl, in which case z is 2, or butyl, in which case z is 4, and addition salts thereof with a pharmaceutically acceptable acid or base.

18. Compounds of formula (I) according to claim 1 which are 6-{2-[4-(2-pyrimidyl)-1-piperazinyl]ethyl}benzothiazolinone, its 3-methyl homologue, and addition salts thereof with a pharmaceutically acceptable acid or base.

19. Compounds of formula (I) according to claim 1 which are 6-{4-[4-(4-trifluoromethylphenyl)-1-piperazinyl]butyl}benzothiazolinone its 3-methyl homologue, and addition salts thereof with a pharmaceutically acceptable acid or base.

20. Compounds of formula (I) according to claim 1 which are 6-{4-[4-(4-fluorophenyl)-1-piperazinyl]butyl}benzothiazolinone its 3-methyl homologue, and addition salts thereof with a pharmaceutically acceptable acid or base.

21. Compounds of formula (I) according to claim 1 which are 7-{2-[4-aryl-1-piperazinyl]ethyl}-2,3-dihydro-3-oxo-ben- zoxazines, their 4-methyl derivatives, with aryl representing a phenyl, 2-methoxyphenyl, 4-fluorophenyl or 3-trifluoromethylphenyl group, and addition salts thereof with a pharmaceutically acceptable acid or base.

22. Compounds of formula (I) according to claim 1 which are 6-{z-[4-phenyl-1-piperazinyl]alkyl}benzothiazolinone, wherein alkyl represents an ethyl group and z is 2, or alkyl represents a butyl group and z is 4, their 3-methyl homologues, and addition salts thereof with a pharmaceutically acceptable acid or base.

23. Process for the preparation of compounds of formula (I) according to claim 1, characterised in that there is used as starting material a compound of formula (ll): 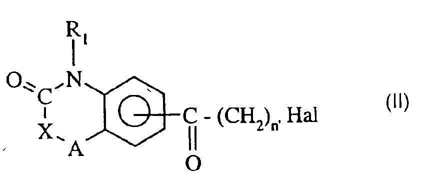 wherein Hal represents a halogen atom, Ry, A and X are as defined in formula (I), and n' represents 1 or 3, which is treated with a trialkylsilane in an acid medium to yield a compound of formula (I II): 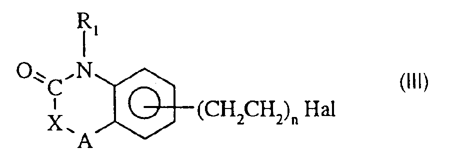 wherein A, X, R₁, n and Hal are as defined above,
which is condensed with an amine of formula (IV): 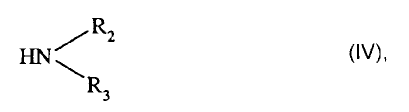 wherein R₂ and R₃ are as defined above, to yield a compound of formula (I): 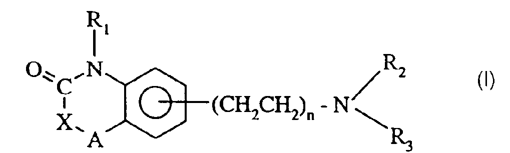 wherein R₁, X, A, n, R₂ and R₃ are as defined above,
the isomers of which are separated, where appropriate, and purified, if necessary, by chromatography or crystallisation,
and which may, if desired, be converted into a salt using a pharmaceutically acceptable acid or base.

24. Pharmaceutical composition comprising as active ingredient at least one compound according to one of claims 1 to 22, in combination with one or more inert, non-toxic, pharmaceutically acceptable excipients or carriers.

25. Pharmaceutical composition according to claim 24 comprising at least one active ingredient according to one of claims 1 to 22, for use as a psychotropic agent, an analgesic or an,antihypertensive.

## Claims (Claims for the following Contracting State(s) : ES, GR)

1. Process for the preparation of compounds of the general formula (I): 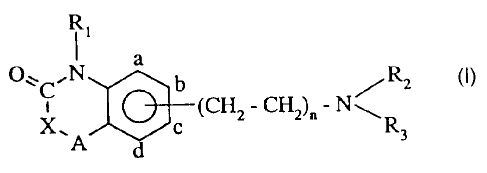 in which:
R₁ represents a hydrogen atom or a lower alkyl group,
n represents 1 or 2,
A represents an oxygen atom or a sulphur atom,
X represents a group CH₂ or a single bond, and
R₂ and R₃ form together with the nitrogen atom carrying them a monocyclic heterocyclic system having six ring members including a second hetero atom selected from nitrogen and oxygen, which heterocyclic system, where appropriate, is unsubstituted or substituted on the optional nitrogen atom by a lower alkyl, phenyl, phenyl-lower alkyl, pyridyl or pyrimidyl group; or by a phenyl group substituted by one or more groups lower alkyl, trifluoromethyl, lower alkoxy, or halogen atoms; or a phenyl-lower alkyl group substituted on the phenyl ring by one or more groups lower alkyl, trifluoromethyl, lower alkoxy, or halogen atoms; or by a pyridyl group substituted by one or more groups lower alkyl, trifluoromethyl, lower alkoxy, or halogen atoms;
wherein:
- when A represents an oxygen atom and X is a group CH₂, the group (CH₂-CH₂)ₙ-NR₂R₃ may not be in position b, and
when A represents an oxygen atom, X is a single bond and n is 1, the group (CH₂-CH₂)ₙ-NR₂R₃ may not be in position c,
and wherein "lower alkyl" is to be understood as meaning linear or branched (C₁-C₆)alkyl,
their enantiomers, diastereoisomers and epimers, and addition salts thereof with a pharmaceutically acceptable acid or a pharmaceutically acceptable base when R₁ is hydrogen,
characterised in that there is used as starting material a compound of formula (II): 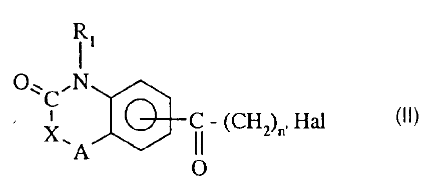 wherein Hal represents a halogen atom, R₁, A and X are as defined in formula (I), and n' represents 1 or 3, which is treated with a trialkylsilane in an acid medium to yield a compound of formula (lll): 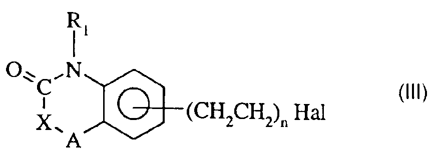 wherein A, X, R₁, n and Hal are as defined above,
which is condensed with an amine of formula (IV): 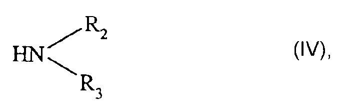 wherein R₂ and R₃ are as defined above, to yield a compound of formula (I): 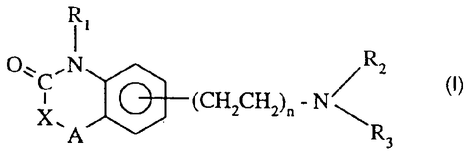 wherein R_{1'} X, A, n, R₂ and R₃ are as defined above,
the isomers of which are separated, where appropriate, and purified, if necessary, by chromatography or crystallisation,
and which may, if desired, be converted into a salt using a pharmaceutically acceptable acid or base.

2. Process according to claim 1 for the preparation of compounds of formula (I) in which X represents a single bond, their enantiomers, epimers and diastereoisomers, and addition salts thereof with a pharmaceutically acceptable acid or a pharmaceutically acceptable base when R₁ is hydrogen.

3. Process according to claim 1 for the preparation of compounds of formula (I) in which X represents a group CH₂, their enantiomers, epimers and diastereoisomers, and addition salts thereof with a pharmaceutically acceptable acid or a pharmaceutically acceptable base when R₁ is hydrogen.

4. Process according to claim 1 for the preparation of compounds of formula (I) in which A represents an oxygen atom and X represents a single bond, their enantiomers, epimers and diastereoisomers, and addition salts thereof with a pharmaceutically acceptable acid or a pharmaceutically acceptable base when R₁ is hydrogen.

5. Process according to claim 1 for the preparation of compounds of formula (I) in which A represents a sulphur atom, their enantiomers, epimers and diastereoisomers, and addition salts thereof with a pharmaceutically acceptable acid or a pharmaceutically acceptable base when R₁ is hydrogen.

6. Process according to claim 1 for the preparation of compounds of formula (I) in which A represents an oxygen atom and X represents a group CH₂, their enantiomers, epimers and diastereoisomers, and addition salts thereof with a pharmaceutically acceptable acid or a pharmaceutically acceptable base when R₁ is hydrogen.

7. Process according to claim 1 for the preparation of compounds of formula (I) in which A represents a sulphur atom and X represents a single bond, their enantiomers, epimers and diastereoisomers, and addition salts thereof with a pharmaceutically acceptable acid or a pharmaceutically acceptable base when R₁ is hydrogen.

8. Process according to claim 1 for the preparation of compounds of formula (I) in which the group (CH₂-CH₂)ₙNR₂R₃ is in position b, their enantiomers, epimers and diastereoisomers, and addition salts thereof with a pharmaceutically acceptable acid or a pharmaceutically acceptable base when R₁ is hydrogen.

9. Process according to claim 1 for the preparation of compounds of formula (I) in which the group (CH₂-CH₂),NR₂R₃ is in position c, their enantiomers, epimers and diastereoisomers, and addition salts thereof with a pharmaceutically acceptable acid or a pharmaceutically acceptable base when R₁ is hydrogen.

10. Process according to claim 1 for the preparation of compounds of formula (I) in which the group (CH₂-CH₂)ₙNR₂R₃ is in position d, their enantiomers, epimers and diastereoisomers, and addition salts thereof with a pharmaceutically acceptable acid or a pharmaceutically acceptable base when R₁ is hydrogen.

11. Process according to claim 1 for the preparation of compounds of formula (I) in which X represents a single bond, A represents a sulphur atom and R₂ and R₃ form with the nitrogen atom carrying them a piperazine that is optionally substituted on the piperazine nitrogen numbered 4 by a phenyl, phenyl-lower alkyl or pyridyl group, the phenyl, phenylalkyl and pyridyl groups themselves being optionally substituted on the aromatic ring by one or more groups lower alkyl, trifluoromethyl, lower alkoxy, or halogen atoms, their enantiomers, epimers and diastereoisomers, and addition salts thereof with a pharmaceutically acceptable acid or a pharmaceutically acceptable base when R₁ is hydrogen.

12. Process according to claim 1 for the preparation of compounds of formula (I) which are 6-{2-(4-(3-trifluoromethylphenyl)piperazin-1-yl]ethyl}benzothiazolinone, its 3-methyl homologue, and addition salts thereof with a pharmaceutically acceptable acid.

13. Process according to claim 1 for the preparation of compounds of formula (I) which are 6-{2-[4-(2-methoxyphenyl)piperazin-1-yl]ethyl}benzothiazolinone, its 3-methyl homologue, and addition salts thereof with a pharmaceutically acceptable acid.

14. Process according to claim 1 for the preparation of compounds of formula (I) which are 6-{2-[4-(4-fluorophenyl)piperazin-1-yl]ethyl}benzothiazolinone, its 3-methyl homologue, and addition salts thereof with a pharmaceutically acceptable acid.

15. Process according to claim 1 for the preparation of compounds of formula (I) which are 6-{4-[4-(2-methoxyphenyl)piperazin-1-yl]butyl}benzothiazolinone, its 3-methyl homologue, and addition salts thereof with a pharmaceutically acceptable acid.

16. Process according to claim 1 for the preparation of compounds of formula (I) which are 7-{4-[4-arylpiperazin-1-yl]butyl}-2,3-dihydro-3-oxo-1,4-benzoxazine, their 4-methyl homologues, with aryl representing a phenyl, 2-methoxyphenyl, 3-trifluoromethylphenyl or 4-fluorophenyl group, and addition salts thereof with a pharmaceutically acceptable acid or base.

17. Process according to claim 1 for the preparation of compounds of formula (I) which are 7-{z-[4-arylpiperazin-1-yl]alkyl}-2,3-dihydro-3-oxo-1,4-benzothiazine, their 4-methyl derivatives, with aryl representing a phenyl, 4-fluorophenyl, 2-methoxyphenyl or 3-trifluoromethylphenyl group, and alkyl representing ethyl, in which case z is 2, or butyl, in which case z is 4, and addition salts thereof with a pharmaceutically acceptable acid or base.

18. Process according to claim 1 for the preparation of compounds of formula (I) which are 6-{2-[4-(2-pyrimidyl)-1-piperazinyl]ethyl}benzothiazolinone, its 3-methyl homologue, and addition salts thereof with a pharmaceutically acceptable acid or base.

19. Process according to claim 1 for the preparation of compounds of formula (I) which are 6-{4-[4-(4-trifluoromethylphenyl)-1-piperazinyl]butyl}benzothiazolinone its 3-methyl homologue, and addition salts thereof with a pharmaceutically acceptable acid or base.

20. Process according to claim 1 for the preparation of compounds of formula (I) which are 6-{4-[4-(4-fluorophenyl)-1-piperazinyl}butyl}benzothiazolinone, its 3-methyl homologue, and addition salts thereof with a pharmaceutically acceptable acid or base.

21. Process according to claim 1 for the preparation of compounds of formula (I) which are 7-{2-[4-aryl-1-piperazinyl]ethyl}-2,3-dihydro-3-oxo-benzoxazines their 4-methyl derivatives, with aryl representing a phenyl, 2-methoxyphenyl, 4-fluorophenyl or 3-trifluoromethylphenyl group, and addition salts thereof with a pharmaceutically acceptable acid or base.

22. Process according to claim 1 for the preparation of compounds of formula (I) which are 6-{z-[4-phenyl-1-piperazinyl]alkyl}benzothiazolinone, wherein alkyl represents an ethyl group and z is 2, or alkyl represents a butyl group and z is 4, their 3-methyl homologues, and addition salts thereof with a pharmaceutically acceptable acid or base.

23. Process for the preparation of pharmaceutical compositions comprising as active ingredient at least one compound obtained according to one of claims 1 to 22, in combination with one or more inert, non-toxic, pharmaceutically acceptable excipients or carriers.

24. Process for the preparation of pharmaceutical compositions according to claim 24 comprising at least one active ingredient obtained according to one of claims 1 to 22, for use as psychotropic agent, analgesic, antihypertensive, or as normolipidaemic agent.

## Patentansprüche (Patentansprüche für folgende(n) Vertragsstaat(en) : AT, BE, CH, DE, DK, FR, GB, IT, LI, LU, NL, SE)

1. Verbindungen der allgemeinen Formel (I): 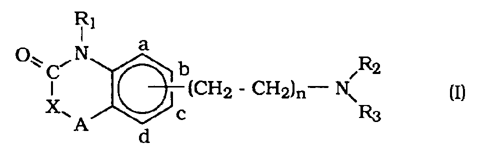 in der
R₁ ein Wasserstoffatom oder eine Niedrigalkylgruppe,
n 1 oder 2,
A ein Sauerstoffatom oder ein Schwefelatom,
X eine CH₂-Gruppe oder eine Einfachbindung und
R₂ und R₃ gemeinsam mit dem sie tragenden Stickstoffatom ein monocyclisches sechsgliedriges heterocyclisches System, welches ein zweites Heteroatom ausgewählt aus Stickstoff oder Sauerstoff enthält und welches gegebenenfalls am Stickstoffatom durch eine Niedrigalkylgruppe, Phenylgruppe, Phenylniedrigalkylgruppe, Pyridylgruppe, Pyrimidylgruppe oder eine durch eine oder mehrere Niedrigalkylgruppen, Trifluormethylgruppen, Niedrigalkoxygruppen oder Halogenatome substituierte Phenylgruppe oder eine am Phenylkern durch eine oder mehrere Niedrigalkylgruppen, Trifluormethylgruppen, Niedrigalkoxygruppen oder Halogenatome substituierte Phenylniedrigalkylgruppe oder eine durch eine oder mehrere Niedrigalkylgruppen, Trifluormethylgruppen, Niedrigalkoxygruppen oder Halogenatome substituierte Pyridylgruppe substituiert ist, mit der Maßgabe bedeuten, daß:
- wenn A ein Sauerstoffatom und X eine CH₂-Gruppe darstellen, die Gruppe der Formel (CH₂-CH₂)-NR₂R₃ nicht in der Stellung b steht,
- wenn A ein Sauerstoffatom, X eine Einfachbindung und n 1 bedeuten, die Gruppe (CH₂-CH₂)_{"}-NR₂R₃ nicht in der Stellung c steht,
wobei unter "Niedrigalkyl" geradkettiges oder verzweigtes (C1-CG)-Alkyl zu verstehen ist,
deren Enantiomere, Diastereoisomere und Epimere sowie deren Additionssalze mit einer pharmazeutisch annehmbaren Säure oder einer pharmazeutisch annehmbaren Base, wenn R₁ = H ist.

2. Verbindungen der Formel (I) nach Anspruch 1, worin X eine Einfachbindung bedeutet, deren Enantiomere, Epimere, Diastereoisomere sowie deren Additionssalze mit einer pharmazeutisch annehmbaren Säure oder einer pharmazeutisch annehmbaren Base, wenn R₁ = H ist.

3. Verbindungen der Formel (I) nach Anspruch 1, worin X eine CH₂-Gruppe darstellt, deren Enantiomere, Epimere, Diastereoisomere sowie deren Additionssalze mit einer pharmazeutisch annehmbaren Säure oder einer pharmazeutisch annehmbaren Base, wenn R₁ = H ist.

4. Verbindungen der Formel (I) nach Anspruch 1, worin A ein Sauerstoffatom und X eine Einfachbindung bedeuten, deren Enantiomere, Epimere, Diastereoisomere sowie deren Additionssalze mit einer pharmazeutisch annehmbaren Säure oder einer pharmazeutisch annehmbaren Base, wenn R₁ = H ist.

5. Verbindungen der Formel (I) nach Anspruch 1, worin A ein Schwefelatom darstellt, deren Enantiomere, Epimere, Diastereoisomere sowie deren Additionssalze mit einer pharmazeutisch annehmbaren Säure oder einer pharmazeutisch annehmbaren Base, wenn R₁ = H ist.

6. Verbindungen der Formel (I) nach Anspruch 1, worin A ein Sauerstoffatom und X eine CH₂-Gruppe bedeuten, deren Enantiomere, Epimere, Diastereoisomere sowie deren Additionssalze mit einer pharmazeutisch annehmbaren Säure oder einer pharmazeutisch annehmbaren Base, wenn R₁ = H ist.

7. Verbindungen der Formel (I) nach Anspruch 1, worin A ein Schwefelatom und X eine Einfachbindung bedeuten, deren Enantiomere, Epimere, Diastereoisomere sowie deren Additionssalze mit einer pharmazeutisch annehmbaren Säure oder einer pharmazeutisch annehmbaren Base, wenn R₁ = H ist.

8. Verbindungen der Formel (I) nach Anspruch 1, worin die Gruppe (CH₂-CH₂)ₙNR₂R₃ in der Stellung b steht, deren Enantiomere, Epimere, Diastereoisomere sowie deren Additionssalze mit einer pharmazeutisch annehmbaren Säure oder einer pharmazeutisch annehmbaren Base, wenn R₁ = H ist.

9. Verbindungen der Formel (I) nach Anspruch 1, worin die Gruppe (CH₂-CH₂)ₙNR₂R₃ in der Stellung c steht, deren Enantiomere, Epimere, Diastereoisomere sowie deren Additionssalze mit einer pharmazeutisch annehmbaren Saure oder einer pharmazeutisch annehmbaren Base, wenn R₁ = H ist.

10. Verbindungen der Formel (I) nach Anspruch 1, worin die Gruppe (CH₂-CH₂)ₙNR₂R₃ in der Stellung d steht, deren Enantiomere, Epimere, Diastereoisomere sowie deren Additionssalze mit einer pharmazeutisch annehmbaren Säure oder einer pharmazeutisch annehmbaren Base, wenn R₁ = H ist.

11. Verbindungen der Formel (I) nach Anspruch 1, worin X eine Einfachbindung, A ein Schwefelatom und R₂ und R₃ zusammen mit dem sie tragenden Stickstoffatom ein Piperazin bilden, welches gegebenenfalls am Piperazin-Stickstoff in der Stellung 4 durch eine Phenylgruppe, Phenylniedrigalkylgruppe oder Pyridylgruppe substituiert ist, wobei die Phenyl-, Phenylalkyl- oder Pyridylgruppen ihrerseits gegebenenfalls am aromatischen Kern durch eine oder mehrere Niedrigalkylgruppen, Trifluormethylgruppen, Niedrigalkoxygruppen oder Halogenatome substituiert sein können, deren Enantiomere, Epimere, Diastereoisomere sowie deren Additionssalze mit einer pharmazeutisch annehmbaren Säure oder einer pharmazeutisch annehmbaren Base, wenn R₁ = H ist.

12. Verbindungen der Formel (I) nach Anspruch 1, nämlich 6-{2-[4-(3-Trifluormethylphenyl)-piperazin-1-yl]-ethyl}-ben- zothiazolinon, dessen 3-Methylhomologes sowie deren Additionssalze mit einer pharmazeutisch annehmbaren Säure.

13. Verbindungen der Formel (I) nach Anspruch 1, nämlich 6-{2-[4-[2-Methoxyphenyl)-piperazin-1-yl] -ethyl}-benzot- hiazolinon, dessen 3-Methylhomologes sowie deren Additionssalze mit einer pharmazeutisch annehmbaren Säure.

14. Verbindungen der Formel (I) nach Anspruch 1, nämlich 6-{2-[4-(4-Fluorphenyl)-piperazin-1-yl]-ethyl}-benzothiazo- linon, dessen 3-Methylhomologes sowie deren Additionssalze mit einer pharmazeutisch annehmbaren Säure.

15. Verbindungen nach Anspruch 1, nämlich 6-{4-[4-(2-Methoxyphenyl)-piperazin-1-yl]-butyl}-benzothiazolinon, dessen 3-Methylhomologes sowie deren Additionssalze mit einer pharmazeutisch annehmbaren Säure.

16. Verbindungen nach Anspruch 1, nämlich 7-{4-[4-Arylpiperazin-1-yl]-butyl}-2,3-dihydro-3-oxo-1,4-benzoxazine, deren 4-Methylhomologe, wobei Aryl für eine Phenyl-, 2-Methoxyphenyl-, 3-Trifiuormethyiphenyt- oder 4-Fluorphenylgruppe steht, sowie deren Additionssalze mit einer pharmazeutisch annehmbaren Säure oder Base.

17. Verbindungen nach Anspruch 1, nämlich 7-{z-[4-Arylpiperazin-1-yl]-alkyll-2,3-dihydro-3-oxo-1,4-benzothiazine, deren 4-Methylderivate, wobei Aryl für eine Phenyl-, 4-Fluorphenyl-, 2-Methoxyphenyl- oder 3-Trifluormethylphenylgruppe steht, Alkyl für eine Ethylgruppe und in diesem Fall z = 2 ist, oder eine Butylgruppe und in diesem Fall z = 4 ist, steht, sowie deren Additionssalze mit einer pharmazeutisch annehmbaren Säure oder Base.

18. Verbindungen der Formel (I) nach Anspruch 1, nämlich 6-{2-[4-[2-Pyrimidyl)-1-piperazinyl]-ethyl}-benzothiazolinon, dessen 3-Methylhomologes sowie deren Additionssalze mit einer pharmazeutisch annehmbaren Säure oder Base.

19. Verbindungen der Formel (I) nach Anspruch 1, nämlich 6-{4-[4-(4-Trifluormethylphenyl)-1-piperazinyl]-butyl}-ben- zothiazolinon, dessen 3-Methylhomologes sowie deren Additionssalze mit einer pharmazeutisch annehmbaren Säure oder Base.

20. Verbindungen der Formel (I) nach Anspruch 1, nämlich 6-{4-[4-(4-Fluorphenyl)-1-piperazinyl]-butyl}-benzothiazoli- non, dessen 3-Methylhomologes sowie deren Additionssalze mit einer pharmazeutisch annehmbaren Säure oder Base.

21. Verbindungen der Formel (I) nach Anspruch 1, nämlich 7-{2-(4-Aryl-1-piperazinyl)-ethyl}-2,3-dihydro-3-oxo-benzoxazine, deren 4-Methylderivate, wobei Aryl für eine Phenyl-, 2-Methoxyphenyl- oder 4-Fluorphenyl- oder 3-Trifluormethylphenylgruppe steht, sowie deren Additionssalze mit einer pharmazeutisch annehmbaren Säure oder Base.

22. Verbindungen der Formel (I) nach Anspruch 1, nämlich 6-{z-[4-Phenyl-1-piperazinyl]-alkyl}-benzothiazolinon, worin Alkyl eine Ethylgruppe und z = 2 oder eine Butylgruppe und z = 4 bedeuten, deren 3-Methylhomologe sowie deren Additionssalze mit einer pharmazeutisch annehmbaren Säure oder Base.

23. Verfahren zur Herstellung der Verbindungen der Formel (I) nach Anspruch 1, dadurch gekennzeichnet, daß man als Ausgangsmaterial ein Derivat der Formel (11) verwendet: 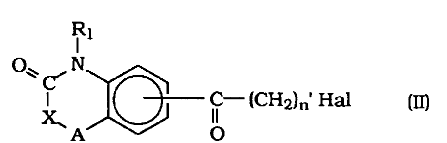 in der Hal ein Halogenatom darstellt und Rᵢ, A, X die bezüglich der Formel (I) angegebenen Bedeutungen besitzen und n' 1 oder 3 bedeutet,
welches manmit einem Trialkylsilan in saurem Medium behandelt zur Bildung eines Derivats der Formel (lll): 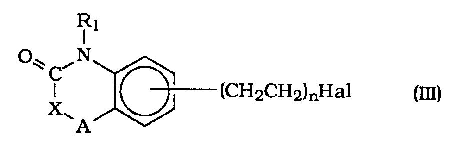 in der A, X, R₁, n und Hal die oben angegebenen Bedeutungen besitzen, welches man mit einem Amin der Formel (IV): 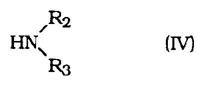 in der R₂ und R₃ die oben angegebenen Bedeutungen besitzen, kondensiert zur Bildung eines Derivats der Formel (I): 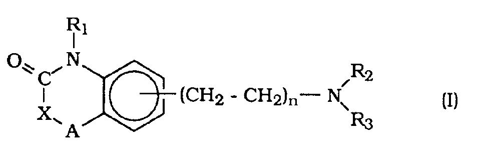 in der R₁, X, A, n, R₂ und R₃ die oben angegebenen Bedeutungen besitzen, welches man gegebenenfalls in die Isomeren auftrennt und erforderlichenfalls durch Chromatographie oder Kristallisation reinigt,
und welches man gewünschtenfalls mit einer pharmazeutisch annehmbaren Säure oder Base In ein Salz überführen kann.

24. Pharmazeutische Zubereitung enthaltend als Wirkstoff mindestens eine Verbindung nach einem der Ansprüche 1 bis 22 in Kombination mit einem oder mehreren inerten, nichttoxischen, pharmazeutisch annehmbaren Trägermaterialien oder Bindemitteln.

25. Pharmazeutische Zubereitung nach Anspruch 24, enthaltend mindestens einen Wirkstoff nach einem der Ansprüche 1 bis 22 zur Verwendung als psychotropes Mittel, analgetisches Mittel oder antihypertensives Mittel.

## Patentansprüche (Patentansprüche für folgende(n) Vertragsstaat(en) : ES, GR)

1. Verfahren zur Herstellung der Verbindungen der allgemeinen Formel (I): 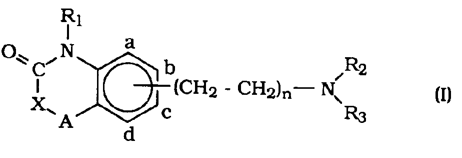 in der
R₁ ein Wasserstoffatom oder eine Niedrigalkylgruppe,
n 1 oder 2,
A ein Sauerstoffatom oder ein Schwefelatom,
X eine CH₂-Gruppe oder eine Einfachbindung und
R₂ und R₃ gemeinsam mit dem sie tragenden Stickstoffatom ein monocyclisches sechsgliedriges heterocyclisches System, welches ein zweites Heteroatom ausgewählt aus Stickstoff oder Sauerstoff enthält und welches gegebenenfalls am Stickstoffatom durch eine Niedrigalkylgruppe, Phenylgruppe, Phenylniedrigalkylgruppe, Pyridylgruppe, Pyrimidylgruppe oder eine durch eine oder mehrere Niedrigalkylgruppen, Trifluormethylgruppen, Niedrigalkoxygruppen oder Halogenatome substituierte Phenylgruppe oder eine am Phenylkern durch eine oder mehrere Niedrigalkylgruppen, Trifluormethylgruppen, Niedrigalkoxygruppen oder Halogenatome substituierte Phenylniedrigalkylgruppe oder eine durch eine oder mehrere Niedrigalkylgruppen, Trifluormethylgruppen, Niedrigalkoxygruppen oder Halogenatome substituierte Pyridylgruppe substituiert ist,
mit der Maßgabe bedeuten, daß:
- wenn A ein Sauerstoffatom und X eine CH₂-Gruppe darstellen, die Gruppe der Formel (CH₂-CH₂)-NR₂R₃ nicht In der Stellung b steht,
- wenn A ein Sauerstoffatom, X eine Einfachbindung und n 1 bedeuten, die Gruppe (CH₂-CH₂)ₙ-NR₂R₃ nicht in der Stellung c steht,
wobei unter "Niedrigalkyl" geradkettiges oder verzweigtes (C₁-C₆)-Alkyl zu verstehen ist,
und von deren Enantiomeren, Diastereoisomeren und Epimeren sowie von deren Additionssalzen mit einer pharmazeutisch annehmbaren Säure oder einer pharmazeutisch annehmbaren Base, wenn R₁ = H bedeutet, dadurch gekennzeichnet, daß man als Ausgangsmaterial ein Derivat der Formel (11) verwendet: 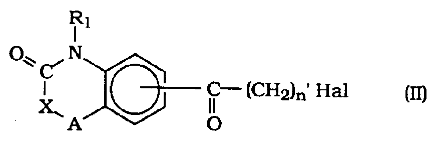 in der Hal ein Halogenatom darstellt und R₁, A, X die bezuglich der Formel (I) angegebenen Bedeutungen besitzen und n' 1 oder 3 bedeutet,
welches man mit einem Trialkylsilan in saurem Medium behandelt zur Bildung eines Derivats der Formel (111): 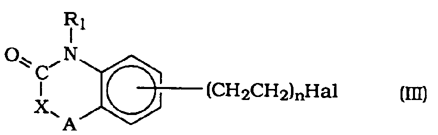 in der A, X, R₁, n und Hal die oben angegebenen Bedeutungen besitzen, welches man mit einem Amin der Formel (IV): 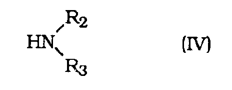 in der R₂ und R₃ die oben angegebenen Bedeutungen besitzen, kondensiert zur Bildung eines Derivats der Formel (I): 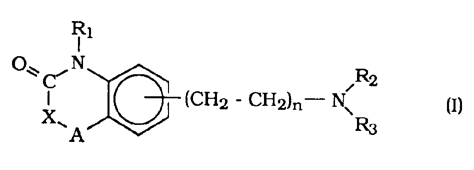 in der R₁, X, A, n, R₂ und R₃ die oben angegebenen Bedeutungen besitzen, welches man gegebenenfalls in die Isomeren auftrennt und erforderlichenfalls durch Chromatographie oder Kristallisation reinigt,
und welches man gewünschtenfalls mit einer pharmazeutisch annehmbaren Säure oder Base in ein Salz überfuhren kann.

2. Verfahren nach Anspruch 1 zur Herstellung der Verbindungen der Formel (I), in der X eine Einfachbindung bedeutet, von deren Enantiomeren, Epimeren, Diastereoisomeren und deren Additionssalzen mit einer pharmazeutisch annehmbaren Säure oder einer pharmazeutisch annehmbaren Base, wenn R₁ = H bedeutet.

3. Verfahren nach Anspruch 1 zur Herstellung der Verbindungen der Formel (I), worin X eine CH₂-Gruppe darstellt, von deren Enantiomeren, Epimeren, Diastereoisomeren sowie von deren Additionssalzen mit einer pharmazeutisch annehmbaren Säure oder einer pharmazeutisch annehmbaren Base, wenn Rᵢ = H ist.

4. Verfahren nach Anspruch 1 zur Herstellung der Verbindungen der Formel (I), worin A ein Sauerstoffatom und X eine Einfachbindung bedeuten, von deren Enantiomeren, Epimeren, Diastereoisomeren sowie von deren Additionssalzen mit einer pharmazeutisch annehmbaren Säure oder einer pharmazeutisch annehmbaren Base, wenn Rᵢ = H ist.

5. Verfahren nach Anspruch 1 zur Herstellung der Verbindungen der Formel (I), worin A ein Schwefelatom darstellt, von deren Enantiomeren, Epimeren, Diastereoisomeren sowie von deren Additionssalzen mit einer pharmazeutisch annehmbaren Säure oder einer pharmazeutisch annehmbaren Base, wenn R₁ = H ist.

6. Verfahren nach Anspruch 1 zur Herstellung der Verbindungen der Formel (I), worin A ein Sauerstoffatom und X eine CH₂-Gruppe bedeuten, von deren Enantiomeren, Epimeren, Diastereoisomeren sowie von deren Additionssalzen mit einer pharmazeutisch annehmbaren Säure oder einer pharmazeutisch annehmbaren Base, wenn R₁ = H ist.

7. Verfahren nach Anspruch 1 zur Herstellung der Verbindungen der Formel (I), worin A ein Schwefelatom und X eine Einfachbindung bedeuten, von deren Enantiomeren, Epimeren, Diastereoisomeren sowie von deren Additionssalzen mit einer pharmazeutisch annehmbaren Säure oder einer pharmazeutisch annehmbaren Base, wenn R₁ = H ist.

8. Verfahren nach Anspruch 1 zur Herstellung der Verbindungen der Formel (I), worin die Gruppe (CH₂-CH₂)ₙNR₂R₃ in der Stellung b steht, von deren Enantiomeren, Epimeren, Diastereoisomeren sowie von deren Additionssalzen mit einer pharmazeutisch annehmbaren Säure oder einer pharmazeutisch annehmbaren Base, wenn R₁ = H ist.

9. Verfahren nach Anspruch 1 zur Herstellung der Verbindungen der Formel (I), worin die Gruppe (CH₂-CH₂)ₙNR₂R₃ in der Stellung c steht, von deren Enantiomeren, Epimeren, Diastereoisomeren sowie von deren Additionssalzen mit einer pharmazeutisch annehmbaren Säure oder einer pharmazeutisch annehmbaren Base, wenn R₁ = H ist.

10. Verfahren nach Anspruch 1 zur Herstellung der Verbindungen der Formel (I), worin die Gruppe (CH₂-CH₂)ₙNR₂R₃ in der Stellung d steht, von deren Enantiomeren, Epimeren. Diastereoisomeren sowie von deren Additionssalzen mit einer pharmazeutisch annehmbaren Säure oder einer pharmazeutisch annehmbaren Base, wenn R₁ = H ist.

11. Verfahren nach Anspruch 1 zur Herstellung der Verbindungen der Formel (I), worin X eine Einfachbindung, A ein Schwefelatom und R₂ und R₃ zusammen mit dem sie tragenden Stickstoffatom ein Piperazin bilden, welches gegebenenfalls am Piperazin-Stickstoff in der Stellung 4 durch eine Phenylgruppe, Phenylniedrigalkylgruppe oder Pyridylgruppe substituiert ist, wobei die Phenyl-, Phenylalkyl- oder Pyridylgruppen ihrerseits gegebenenfalls am aromatischen Kern durch eine oder mehrere Niedrigalkylgruppen, Trifluormethylgruppen, Niedrigalkoxygruppen oder Halogenatome substituiert sein können, von deren Enantiomeren, Epimeren, Diastereoisomeren sowie von deren Additionssalzen mit einer pharmazeutisch annehmbaren Säure oder einer pharmazeutisch annehmbaren Base, wenn R₁ = H ist.

12. Verfahren nach Anspruch 1 zur Herstellung der Verbindungen der Formel (I), nämlich 6-{2-[4-(3-Trifluormethylphenyl)-piperazin-1-yl]-ethyl}-benzothiazolinon, von dessen 3-Methylhomologem sowie von dessen Additionssalzen mit einer pharmazeutisch annehmbaren Säure.

13. Verfahren nach Anspruch 1 zur Herstellung der Verbindungen der Formel (I), nämlich 6-{2-[4-(2-Methoxyphenyl)-piperazin-1-yl]-ethyl}-benzothiazolinon, von dessen 3-Methylhomologem sowie von dessen Additionssalzen mit einer pharmazeutisch annehmbaren Säure.

14. Verfahren nach Anspruch 1 zur Herstellung der Verbindungen der Formel (I), nämlich 6-{2-[4-(4-Fluorphenyl)-piperazin-1-yl]-ethyl}-benzothiazolinon, von dessen 3-Methylhomologem sowie von dessen Additionssalzen mit einer pharmazeutisch annehmbaren Säure.

15. Verfahren nach Anspruch 1 zur Herstellung der Verbindungen der Formel (I), nämlich 6-{4-[4-(2-Methoxyphenyl)-piperazin-1-yl]-butyl}-benzothiazolinon, von dessen 3-Methylhomologem sowie von dessen Additionssalzen mit einer pharmazeutisch annehmbaren Säure.

16. Verfahren nach Anspruch 1 zur Herstellung der Verbindungen der Formel (I), nämlich 7-{4-[4-Arylpiperazin-1-yl]-butyl}-2,3-dihydro-3-oxo-1 ,4-benzoxazin, von deren 4-Methylhomologen, wobei Aryl für eine Phenyl-, 2-Methoxyphenyl-, 3-Trifluormethylphenyl- oder 4-Fluorphenylgruppe steht, sowie von dessen Additionssalzen mit einer pharmazeutisch annehmbaren Säure oder Base.

17. Verfahren nach Anspruch 1 zur Herstellung der Verbindungen der Formel (I), nämlich 7-{z-[4-Arylpiperazin-1-yl]-alkyl}-2,3-dihydro-3-oxo-1,4-benzothiazin, von deren 4-Methylderivaten, wobei Aryl für eine Phenyl-, 4-Fluorphenyl-, 2-Methoxyphenyl oder 3-Trifluormethylphenylgruppe steht, Alkyl für eine Ethylgruppe und in diesem Fall z = 2 ist, oder eine Butylgruppe und in diesem Fall z = 4 ist, steht, sowie von dessen Additionssalzen mit einer pharmazeutisch annehmbaren Säure oder Base.

18. Verfahren nach Anspruch 1 zur Herstellung der Verbindungen der Formel (I), nämlich 6-{2-[4-(2-Pyrimidyl)-1-piperazinyl]-ethyl}-benzothiazolinon, von dessen 3-Methylhomologem sowie von dessen Additionssalzen mit einer pharmazeutisch annehmbaren Säure oder Base.

19. Verfahren nach Anspruch 1 zur Herstellung der Verbindungen der Formel (I), nämlich 6-{4-[4-(4-Trifluormethylphenyl)-1-piperazinyl]-butyl}-benzothiazolinon, von dessen 3-Methylhomologem sowie von dessen Additionssalzen mit einer pharmazeutisch annehmbaren Säure oder Base.

20. Verfahren nach Anspruch 1 zur Herstellung der Verbindungen der Formel (I), nämlich 6-{4-[4-(4-Fluorphenyl)-1-piperazinyl]-butyl}-benzothiazolinon, von dessen 3-Methylhomologem sowie von dessen Additionssalzen mit einer pharmazeutisch annehmbaren Säure oder Base.

21. Verfahren nach Anspruch 1 zur Herstellung der Verbindungen der Formel (I), nämlich 7-{2-(4-Aryl-1-piperazinyl)-ethyl}-2,3-dihydro-3-oxo-benzoxazin, von deren 4-Methylderivaten, wobei Aryl für eine Phenyl-, 2-Methoxyphenyl-oder 4-Fluorphenyl- oder 3-Trifluormethylphenylgruppe steht, sowie von deren Additionssalzen mit einer pharmazeutisch annehmbaren Säure oder Base.

22. Verbindungen der Formel (I) nach Anspruch 1, nämlich 6-{z-[4-Phenyl-1-piperazinyl]-alkyl}-benzothiazolinon, worin Alkyl eine Ethylgruppe und z = 2 oder eine Butylgruppe und z = 4 bedeuten, deren 3-Methylhomologe sowie deren Additionssalze mit einer pharmazeutisch annehmbaren Säure oder Base.

23. Verfahren zur Herstellung von pharmazeutischen Zubereitungen enthaltend als Wirkstoff mindestens eine Verbindung erhalten nach einem der Ansprüche 1 bis 22 in Kombination mit einem oder mehreren inerten, nichttoxischen, pharmazeutisch annehmbaren Trägermaterialien oder Bindemitteln.

24. Verfahren zur Herstellung von pharmazeutischen Zubereitungen nach Anspruch 24, enthaltend mindestens einen Wirkstoff erhalten nach einem der Ansprüche 1 bis 22 zur Verwendung als psychotropes Mittel, analgetisches Mittel, antihypertensives Mittel und normolipämisches Mittel.
